# FASCICULE DE BREVET EUROPEEN

(11) **EP 3 687 493 B1**
(45) Date de publication et mention de la délivrance du brevet: **29.12.2021**
(21) Numéro de dépôt: 18752605.8
(22) Date de dépôt: 30.07.2018
(51) Int. Cl.: A61K 9/46, A61K 9/20, A61K 36/185, A61P 1/04

(54) **COMPOSITION ADAPTÉE POUR FORMER UN RADEAU GASTRIQUE GÉLIFIÉ**
ZUSAMMENSETZUNG ZUR BILDUNG EINES GASTRISCHEN GEL-RAFTS
COMPOSITION SUITABLE FOR FORMING A GEL GASTRIC RAFT

(30) Priorité: 29.09.2017 FR 1759155
(43) Date de publication de la demande: 05.08.2020
(73) Titulaire: Laboratoires Arkopharma, 06510 Carros (FR)
(72) Inventeur: GARDIER, Sylvie, 06670 Colomars (FR); COLL, Richard, 06560 Valbonne (FR)
(74) Mandataire: Murgitroyd & Company
(86) Numéro de dépôt international: PCT/FR2018/051955
(87) Numéro de publication internationale: WO 2019/063891

(56) Documents cités:
- WO-A1-95/11668
- David Kiefer ET AL: "An Integrative Approach to GERD (Gastroesophageal Reflux Disease)", , 1 février 2012 (2012-02-01), pages 1-10, XP055481642, University of Wisconsin Extrait de l'Internet: URL:http://www.fammed.wisc.edu/files/webfm -uploads/documents/outreach/im/module_gerd _clinician.pdf [extrait le 2018-06-06]

## Description

### Domaine technique

L'invention concerne le domaine des compositions (par exemple pharmaceutiques ou nutritionnelles) administrables *per os* et adaptées pour :
- traiter ou prévenir les affections gastro-œsophagiennes et en particulier le reflux gastro-œsophagien (RGO) et/ou ses symptômes (tels que les remontées acides, les brûlures d'estomac, la digestion difficile, les indigestions, le pyrosis), la gastrite, la dyspepsie et les ulcères peptidiques,
- permettre la délivrance prolongée ou ciblée d'ingrédient(s) actif(s) dans l'estomac et/ou à la jonction gastro-oesophagienne d'un individu humain ou animal (de préférence humain), et/ou
- permettre le soulagement, au niveau local, des muqueuses gastro-oesophagiennes irritées.

### Etat de la technique

Comme cela est connu dans l'état de la technique, le reflux gastro-œsophagien (RGO) survient lorsqu'une partie du contenu gastrique (suc gastrique, nourriture et/ou acides biliaires) passe dans la partie inférieure de l'œsophage et provoque une inflammation œsophagienne accompagnée de douleurs qui peuvent se manifester sous forme de brûlures d'estomac. Dans certains cas, un reflux gastro-œsophagien excessivement fréquent et/ou prolongé peut être responsable de complications, définissant, quel que soit l'âge du sujet atteint, un reflux gastro-œsophagien (RGO) pathologique, également dénommé maladie du reflux gastro-œsophagien ou, plus simplement, « maladie du reflux » (encore dénommé « Gastro-oesophageal Reflux Disease », en langue anglaise, dont l'acronyme est GORD ou GERD).

Une approche répandue pour traiter ou prévenir le reflux gastro-œsophagien (RGO) et ses symptômes consiste à administrer, à un individu souffrant de reflux gastro-œsophagien, une préparation pharmaceutique qui, en contact avec l'acide gastrique, génère une mousse ou un radeau gélatineux gazéifié (contenant du dioxyde de carbone) qui flotte à la surface du contenu de l'estomac. La résistance de ce radeau en fait une barrière relativement efficace contre le reflux gastro-œsophagien. De plus, en cas de reflux sévère, ce radeau, plus léger que le contenu gastrique, va refluer en premier dans l'œsophage (à savoir précède le contenu de l'estomac dans l'œsophage) pour former un écran de protection au niveau de la muqueuse œsophagienne, protégeant cette dernière contre toute irritation, et notamment au niveau de la jonction gastro-œsophagienne. Les préparations pharmaceutiques de ce type comprennent des préparations pharmaceutiques sous forme solide ou liquide.

Certaines préparations sous forme solide (commercialisées sous le nom de marque GAVISCON^{®}) comprennent de l'acide alginique, du bicarbonate de sodium et du carbonate de calcium. L'acide alginique réagit avec le bicarbonate de sodium dans la cavité buccale, au contact de la salive, pour former une mousse d'alginate (piégeant en son sein le dioxyde de carbone également produit par la réaction), laquelle est ensuite avalée (avec plus au moins de difficultés).

Des préparations liquides comprenant de l'alginate de sodium (en lieu et place de l'acide alginique), du bicarbonate de sodium ou de potassium et du carbonate de calcium ont également été développées. De telles préparations liquides sont commercialisées sous les noms de marque GAVISCON^{®} et GAVISCON^{®} ADVANCE et sont décrites dans les demandes de brevet GB-A-1,524,740 et WO 95/11668.

Les préparations pharmaceutiques solides ou liquides mentionnées supra reposent sur un mécanisme d'action similaire, largement décrit dans la littérature et brièvement résumé ci-après :
- l'alginate de sodium, hydrosoluble, devient de l'acide alginique, non-hydrosoluble sous l'effet du milieu acide de l'estomac (pH<2) et partiellement des ions alginates,
- le carbonate de calcium se dissout dans le contenu stomacal en libérant des ions calcium Ca²⁺ (et du dioxyde de carbone),
- L'acide alginique non hydrosoluble est réticulé par les ions Ca²⁺ (provenant du carbonate de calcium ; les ions Ca²⁺ jouant le rôle d'agents de réticulation de l'acide alginique) produisant ainsi un radeau alginate (la réticulation par les ions Ca²⁺ se produit entre deux chaînes de l'acide alginique appelées « blocs G » [« G-blocks », en langue anglaise], correspondant à des enchaînements de monomères (1 → 4) α-L-guluronate (G), dans la mesure où ces blocs G ont une forme de zigzag qui permet aux ions Ca²⁺ d'établir une liaison ionique avec deux de ces régions riches en monomères G ; la réticulation de ces régions riches en monomères G par les ions Ca²⁺ produit des régions en forme de « boîtes à œufs » [« egg-box », en langue anglaise]),
- Le bicarbonate de sodium (ou de potassium) et le carbonate de calcium réagissent avec le pH acide de l'estomac en donnant lieu à un dégagement de dioxyde de carbone (CO₂), lequel dioxyde de carbone se trouve piégé au sein du « radeau gastrique» (gel visqueux d'acide alginique contenant, en son sein, du CO₂), ce qui a pour effet de faire « flotter » ce radeau alginate à la surface du contenu gastrique,
- Les ions carbonate et bicarbonate formés agissent aussi partiellement en augmentant le pH gastrique.

Même si de telles préparations pharmaceutiques sous forme solide ou liquide (de type GAVISCON^{®}) sont connues et utilisées depuis des décennies, l'analyse de ces préparations pharmaceutiques a révélé que des améliorations significatives étaient nécessaires, en particulier concernant tout ou partie des propriétés suivantes :
i) volume, « flottabilité », et stabilité dans le temps du radeau formé dans l'estomac,
ii) reproductibilité/répétabilité de la formation du radeau,
iii) certaines propriétés rhéologiques du radeau et notamment en termes de tenue (écoulement, consistance et viscosité du radeau).

David Kiefer ET AL: "An Integrative Approach to GERD (Gastroesophageal Reflux Disease)", divulgue que l'écorce d'orme rouge permet de traiter les reflux gastro-oesophagiques.

De manière surprenante, les inventeurs ont découvert, contre toute attente, que l'ajout d'une quantité efficace d'une préparation à base d'écorce d'orme rouge *(Ulmus rubra* Muhl. syn. *Ulmus fulva* Michx. ; « slippery elm », en langue anglaise) - et en particulier à base d'écorce interne d'orme rouge - dans une préparation (par exemple pharmaceutique ou nutritionnelle) sous forme liquide ou solide à base d'acide alginique ou d'alginate, d'au moins un composé produisant un dégagement de dioxyde de carbone en milieu acide et éventuellement d'au moins un agent de réticulation de l'acide alginique (préparation pharmaceutique de type GAVISCON^{®}) permettait d'améliorer substantiellement tout ou partie des propriétés susvisées.

### Exposé de l'invention

Par conséquent, l'invention a pour objet une composition (par exemple à visée pharmaceutique ou nutritionnelle) administrable par voie orale à un individu humain ou animal, adaptée pour former, après ingestion, un radeau gélifié (pouvant également être qualifié de « gel moussant ») flottant à la surface du contenu gastrique dudit individu, ladite composition comprenant (ou consistant essentiellement en) les ingrédients actifs suivants en quantités efficaces :
a) une préparation à base d'écorce d'orme rouge *(Ulmus rubra Muhl.),* de préférence à base d'écorce interne d'orme rouge,
b) de l'acide alginique ou de l'alginate,
c) au moins un composé produisant un dégagement de dioxyde de carbone en milieu acide, tel que :
   c.1) au moins un sel de bicarbonate, de préférence au moins un bicarbonate de métal alcalin, avantageusement du bicarbonate de sodium (NaHCO₃), du bicarbonate de potassium (KHCO₃) ou un mélange de ceux-ci, et/ou
   c.2) au moins un sel de carbonate, de préférence au moins un carbonate de cation métallique divalent ou trivalent, préférablement au moins un carbonate de métal alcalino-terreux, avantageusement du carbonate de calcium (CaCO₃) du carbonate de magnésium (MgCO₃) ou un mélange de ceux-ci, et
d) éventuellement au moins un agent de réticulation de l'acide alginique, de préférence au moins une source de cations métalliques divalents ou trivalents, préférablement au moins un sel de métal alcalino-terreux, avantageusement au moins un sel de calcium (par exemple du carbonate de calcium), un sel de magnésium (par exemple du carbonate de magnésium) ou un mélange des deux.

Tel qu'indiqué supra, les ingrédients actifs susvisés sont présents en quantités efficaces, à savoir dans des quantités/proportions permettant de former, après ingestion, un radeau gélifié flottant à la surface du contenu gastrique du susdit individu humain ou animal. Les quantités/proportions des ingrédients actifs susvisés permettant d'obtenir ceci pourront être déterminées par l'homme du métier sans difficulté excessive sur la base du contenu de la présente demande de brevet et, au besoin, en mettant en oeuvre des tests de routine. De tels tests de routine pourront par exemple consister à déterminer les différentes quantités/proportions d'ingrédients actifs permettant d'observer la formation, *in vitro,* du susdit radeau gélifié, en se plaçant dans des conditions proches, similaires ou identiques aux conditions physiologiques (par exemple en se plaçant en milieu liquide, à pH inférieur à 3 [ou à 2] et à une température d'environ 37°C). Ainsi, l'invention peut également être considérée comme une composition administrable par voie orale à un individu humain ou animal, adaptée pour former, après ingestion, un radeau gélifié flottant à la surface du contenu gastrique dudit individu, ladite composition comprenant (ou consistant essentiellement en) les ingrédients actifs suivants :
a) une préparation à base d'écorce d'orme rouge *(Ulmus rubra Muhl.),* de préférence à base d'écorce interne d'orme rouge,
b) de l'acide alginique ou de l'alginate,
c) au moins un composé produisant un dégagement de dioxyde de carbone en milieu acide, tel que :
   c.1) au moins un sel de bicarbonate, de préférence au moins un bicarbonate de métal alcalin, avantageusement du bicarbonate de sodium, du bicarbonate de potassium ou un mélange de ceux-ci, et/ou
   c.2) au moins un sel de carbonate, de préférence au moins un carbonate de cation métallique divalent ou trivalent, préférablement au moins un carbonate de métal alcalino-terreux, avantageusement du carbonate de calcium, du carbonate de magnésium ou un mélange de ceux-ci, et
d) éventuellement au moins un agent de réticulation de l'acide alginique, de préférence au moins une source de cations métalliques divalents ou trivalents, préférablement au moins un sel de métal alcalino-terreux, avantageusement au moins un sel de calcium, un sel de magnésium ou un mélange des deux,
dans des proportions permettant d'obtenir la formation dudit radeau gélifié dans un milieu liquide ayant un pH inférieur à 3 et à une température d'environ 37°C, le radeau gélifié ainsi formé flottant à la surface dudit liquide.

De préférence, la composition selon l'invention comprend c.1) et/ou c.2).

Avantageusement, la composition selon l'invention comprend l'ingrédient actif d) lorsque :
- c.2) est absent, ou
- c.2) est présent mais est différent d'au moins un carbonate de cation métallique divalent ou trivalent.

Dans un mode de réalisation particulièrement préféré, c) comprend, consiste essentiellement en, ou consiste en c.1) et c.2).

Selon un mode de réalisation particulier, lorsque la composition selon l'invention est sous forme liquide, c.1) est du bicarbonate de potassium ou un mélange bicarbonate de sodium/bicarbonate de potassium.

De préférence, b) est un sel de métal alcalin ou alcalino-terreux d'alginate, de préférence un sel de métal alcalin d'alginate, préférablement un alginate de sodium (n° CAS : 9005-38-3) et/ou un alginate de potassium (n° CAS : 9005-36-1), avantageusement un alginate de sodium.

De préférence, concernant les formes galéniques solides (comme par exemple les comprimés à croquer), l'on utilise, en tant que composé b), un alginate de sodium AL-1 possédant une forte proportion en résidus G. Cet alginate de sodium AL-1 possède une viscosité :
i) comprise entre 20 et 80, de préférence entre 30 et 70 et de manière préférée entre 35 et 65 centipoises dans une solution aqueuse à 1% (poids/volume), en utilisant un viscosimètre de Brookfield (spindle #1) à une vitesse de rotation de 30 t/min (30 rpm) et à une température de 20°C; et/ou
ii) comprise entre 5000 et 20000, de préférence entre 6000 et 16000 et de manière préférée entre 7000 et 14000 centipoises dans une solution aqueuse à 6% (poids/volume), en utilisant un viscosimètre de Brookfield (spindle #3) à une vitesse de rotation de 3 t/min (3 rpm) et à une température de 20°C.

Avantageusement, ledit alginate de sodium AL-1 présente les paramètres de viscosité définis aux points i) et ii) supra.

Selon un mode de réalisation préféré, cet alginate de sodium AL-1 possède en outre au moins un, de préférence deux et avantageusement trois, des paramètres suivants :
- un pH en solution aqueuse à 1 % (poids/volume) compris entre 6 et 8,
- une taille de particules définie comme suit : 95% min (minimum) à travers une maille de « 35 Mesh/425µm (« 95% min through 35 Mesh/ 425µm », en langue anglaise), et
- résistance du gel ("Gel strength", en langue anglaise) : 50g min.

En ce qui concerne les formes galéniques liquides (sirops, suspensions buvables, formes visqueuses etc.), l'on utilise préférablement en tant que composé b), un alginate de sodium AL-2 possédant une viscosité comprise entre 200 et 800, avantageusement entre 300 et 700, centipoises dans une solution aqueuse à 10% (poids/volume) et à une température de 20°C.

Selon un mode de réalisation préféré, l'alginate de sodium AL-2 possède en outre au moins un, de préférence deux et avantageusement trois, des paramètres suivants :
- un pH en solution aqueuse à 1 % (poids/volume) compris entre 6 et 8, et
- une taille de particules définie comme suit : 95% min à travers une maille de « 80 Mesh/180µm (« 95% min through 80 Mesh/180µm», en langue anglaise).

Selon un mode de réalisation de l'invention, les formes galéniques liquides susvisées peuvent comprendre un mélange des alginates de sodium AL-1 et AL-2 susvisés.

Selon un mode de réalisation préféré, le rapport en masse a)/b) est compris entre 0,003 et 12.

Lorsque la composition selon l'invention est sous forme solide (par exemple sous forme de comprimé à croquer), le susdit rapport en masse a)/b) est de préférence compris entre 0,1 et 5, avantageusement entre 0,5 et 3, de préférence entre 1 à 2, et de manière particulièrement préférée entre 1,1 et 1,6.

Lorsque la composition selon l'invention est sous forme liquide, le susdit rapport en masse a)/b) est de préférence compris entre 0,005 et 0,1, préférablement entre 0,01 et 0,05, avantageusement entre 0,01 et 0,03 ; de manière particulièrement préférée ledit rapport en masse étant d'environ 0,02.

Selon un mode de réalisation préféré, le rapport en masse a)/c.1) est compris entre 0,005 et 12.

Lorsque la composition selon l'invention est sous forme solide, le susdit rapport en masse a)/c.1) est de préférence compris entre 0,1 à 5, avantageusement entre 0,5 et 3, de préférence entre 0,6 et 2, et de manière particulièrement préférée entre 0,7 et 1,6.

Lorsque la composition selon l'invention est sous forme liquide, le susdit rapport en masse a)/c.1) est de préférence compris entre 0,005 et 0,1, préférablement entre 0,01 et 0,08, avantageusement entre 0,02 et 0,06 ; de manière particulièrement préférée, ledit rapport en masse étant d'environ 0,04.

Selon un mode de réalisation préféré, le rapport en masse a)/c.2) est compris entre 0,003 et 12.

Lorsque la composition selon l'invention est sous forme solide, le susdit rapport en masse a)/c.2) est de préférence compris entre 0,1 à 5, avantageusement entre 0,5 et 3, de préférence entre 1 à 2, et de manière particulièrement préférée entre 1,2 et 1,6.

Lorsque la composition selon l'invention est sous forme liquide, le susdit rapport en masse a)/c.2) est de préférence compris entre 0,005 et 0,1, préférablement entre 0,01 et 0,09, avantageusement entre 0,03 et 0,08 ; de manière particulièrement préférée, ledit rapport en masse étant d'environ 0,06.

Selon un mode de réalisation, la composition selon l'invention comprend uniquement, en tant qu'ingrédients actifs, les ingrédients actifs a), b), c.1) et c.2).

Selon un mode de réalisation particulièrement préféré, la composition selon l'invention comprend (ou consiste essentiellement en) les ingrédients actifs a), b), c.1) et c.2), et ladite composition comprend, par unité de prise/dose :
- en tant qu'ingrédient actif a) : de la poudre d'écorce d'orme rouge, de préférence d'écorce interne d'orme rouge, avantageusement dans une quantité comprise entre 5 et 600 mg,
- en tant qu'ingrédient actif b) : de l'alginate de sodium, de préférence dans une quantité comprise entre 50 et 1500 mg,
- en tant qu'ingrédient actif c.1) du bicarbonate de sodium, de préférence dans une quantité comprise entre 50 et 1000 mg,
- en tant qu'ingrédient actif c.2) du carbonate de calcium, de préférence dans une quantité comprise entre 50 et 500 mg.

Selon un mode de réalisation particulièrement préféré, le bicarbonate de sodium et le carbonate de calcium sont d'origine minérale (et non issus de la chimie de synthèse), pour permettre à la composition selon l'invention d'être en conformité avec les exigences de « naturalité » posées par la Demanderesse.

Selon un aspect de l'invention, et en particulier lorsque la composition selon l'invention est sous forme solide (par exemple sous forme de comprimé à croquer), le pourcentage massique de l'ingrédient actif a) est compris entre 5 et 20%, de préférence entre 8 et 16%, avantageusement entre 9 et 15%, par rapport à la masse totale de la composition.

Selon un mode de réalisation préféré de cet aspect de l'invention :
- le pourcentage massique de l'ingrédient actif a) est compris entre 5 et 20%, de préférence entre 8 et 16%, avantageusement entre 9 et 15%, par rapport à la masse totale de la composition,
- le pourcentage massique de l'ingrédient actif b) est compris entre 5 et 15%, de préférence entre 7 et 12%, avantageusement entre 7,5 et 10%, par rapport à la masse totale de la composition,
- le pourcentage massique de l'ingrédient actif c.1) est compris entre 10 et 20%, de préférence entre 12 et 16% par rapport à la masse totale de la composition, et
- le pourcentage massique de l'ingrédient actif c.2) est compris entre 5 et 15%, de préférence entre 7 et 12%, avantageusement entre 7,5 et 10%, par rapport à la masse totale de la composition,

Selon un mode de réalisation, la composition selon l'invention comprend les ingrédients actifs a), b), c.1) et c.2), et ladite composition comprend :
- en tant qu'ingrédient actif a) : de la poudre d'écorce d'orme rouge, de préférence d'écorce interne d'orme rouge, avantageusement dans une quantité comprise entre 5 et 600 mg,
- en tant qu'ingrédient actif b) : de l'alginate de sodium, de préférence dans une quantité comprise entre 50 et 1500 mg,
- en tant qu'ingrédient actif c.1) du bicarbonate de sodium, de préférence dans une quantité comprise entre 50 et 1000 mg,
- en tant qu'ingrédient actif c.2) du carbonate de calcium, de préférence dans une quantité comprise entre 50 et 500 mg.

Concernant les formes galéniques solides, la composition selon l'invention se présente avantageusement sous forme de comprimé à croquer. De préférence, ce comprimé à croquer a une masse allant de 1 gramme à 3 grammes, préférablement de 1,5 grammes à 2,5 grammes, avantageusement de 1,7 grammes à 2,3 grammes, et de manière particulièrement préférée de 1,7 grammes à 2 grammes.

L'invention a également pour objet les formes galéniques solides (telles que des comprimés à croquer, des pastilles etc.) et les formes galéniques liquides (solutions, suspensions buvables, gels, sirop etc.) comprenant, consistant essentiellement en, ou consistant en la composition selon l'invention, lesdites formes galéniques liquides étant avantageusement conditionnées en sachet (de préférence hermétique) ou en stick (de préférence hermétique), en particulier pour des raisons de praticité d'administration et pour favoriser l'observance du traitement/de la cure.

Il convient de noter que les formes galéniques liquides (plus simplement dénommées « formes liquides ») s'entendent, au sens large, de toute composition ou formulation susceptible de prendre la forme du contenant qui la contient (par exemple la forme du sachet ou du stick mentionnés ci-dessus) mais dont le volume est déterminé. Ainsi, et tel qu'indiqué supra, ces formes liquides incluent/englobent notamment les solutions, suspensions buvables, gels, sirops.

L'invention a également pour objet la composition selon l'invention pour son utilisation en tant que médicament.

Un autre objet de l'invention concerne la composition selon l'invention pour :
i) le traitement ou la prévention des affections gastro-œsophagiennes et en particulier du reflux gastro-œsophagien (RGO) et/ou ses symptômes (tels que les remontées acides, les brûlures d'estomac la digestion difficile, les indigestions, le pyrosis), de la gastrite, de la dyspepsie et des ulcères peptidiques,
ii) permettre la délivrance prolongée ou ciblée d'ingrédient(s) actif(s) dans l'estomac et/ou à la jonction gastro-œsophagienne dudit individu, et/ou
iii) permettre le soulagement, au niveau local, des muqueuses gastro-œsophagiennes irritées.

L'invention concerne également l'utilisation d'une quantité efficace d'une préparation à base d'écorce d'orme rouge *(Ulmus rubra Muhl.),* de préférence à base d'écorce interne d'orme rouge, d'une quantité efficace d'acide alginique ou d'alginate (par exemple d'alginate de sodium), d'une quantité efficace d'au moins un composé produisant un dégagement de dioxyde de carbone en milieu acide (par exemple du bicarbonate de sodium), et éventuellement au moins un agent de réticulation de l'acide alginique (par exemple du carbonate de calcium), pour préparer une composition adaptée pour former, après administration orale à un individu humain ou animal, un radeau gélifié flottant à la surface du contenu gastrique dudit individu.

Avantageusement, la susdite préparation à base d'écorce d'orme rouge est une poudre d'écorce d'orme rouge, un extrait aqueux d'écorce d'orme rouge et/ou un extrait hydroglycériné d'écorce d'orme rouge, avantageusement une poudre d'écorce d'orme et/ou un extrait aqueux d'écorce d'orme rouge, de manière préférée une poudre d'écorce d'orme rouge, et de manière particulièrement préférée une poudre d'écorce interne d'orme rouge.

Selon un mode de réalisation, la composition selon l'invention peut en outre comprendre un ou plusieurs ingrédient(s) actif(s) additionnel(s). A titre d'exemple, ladite composition peut comprendre une ou plusieurs préparations à base de plantes, de préférence à base de plantes ou d'algues contenant des mucilages. A titre illustratif, l'on peut citer des extraits de plantes ou d'algues de type poudre de plante, extrait aqueux, extrait hydroalcoolique ou extrait hydroglycériné. Par exemple, les extraits aqueux ou hyroglycérinés des plantes suivantes peuvent être inclus dans la composition selon l'invention : Aloe, Althaea officinalis, Commiphora molmol, Fucus vesiculosus, Glycyrrhiza glabra, et Ispaghul.

La composition selon l'invention peut également comprendre une ou plusieurs gommes, et en particulier les gommes sélectionnées parmi : arabinogalactanes (provenant par exemple de Adhatoda vasica et/ou Larix, Withania somnifera), des gommes de type Acacia, Cassia, Gellane, Guar, Karaya, Tamarind, Tara, Konjac, Xanthane, Locust bean.

La composition selon l'invention peut également comprendre un ou plusieurs polymères à propriétés adhésives, en particulier ceux choisis parmi : carboxyméthylcellulose, chitosan, gélatine, acide hyaluronique et dérivés, carbopol, ou polycarbophile, ou d'autres composés polymères à propriétés adhésives.

En outre, la composition selon l'invention peut également comprendre un ou plusieurs arômes, comme par exemple les arômes suivants : menthe, citron, orange, agrumes, fruits rouges, miel.

Lorsque la composition selon l'invention se présente sous forme solide (par exemple sous forme de comprimé à croquer), ladite composition peut comprendre :
- comme agent(s) de charge forme solide : Sorbitol, Isomalt, Maltitol, Mannitol, Saccharose, Phosphate bicalcique, Dextrose,
- comme édulcorant forme solide : sucralose, acésulfame de K, neohespéridine,
- comme lubrifiant forme solide : Stéarine, acide stéarique, HPMC, HPC, amidon et talc.

Selon un mode de réalisation préféré, en particulier lorsque la composition selon l'invention est sous forme liquide, la composition selon l'invention est dépourvue de conservateurs « chimiques » (produits de synthèse d'origine non-naturelle), comme par exemple des conservateurs de type sorbate de potassium, benzoate de sodium ou nipagin, nipasol. Avantageusement ladite composition est également dépourvue d'agents texturants « chimiques » (produits de synthèse d'origine non-naturelle). Ceci confère une originalité additionnelle à cette forme liquide.

Selon un mode de réalisation particulier, la composition selon l'invention peut toutefois comprendre, notamment lorsqu'elle est sous forme liquide, au moins un conservateur de type sorbate de potassium, benzoate de sodium ou nipagin, nipasol et/ou au moins un agent texturant.

Tel qu'indiqué supra, les inventeurs ont découvert, de manière surprenante, que la composition selon l'invention permettait d'améliorer tout ou partie des propriétés intrinsèques au radeau gastrique formé au contact du contenu gastrique acide, et en particulier d'améliorer tout ou partie des propriétés i) - iii) susvisées, lesquelles sont rappelées ci-après :
i) volume, « flottabilité », et stabilité dans le temps du radeau formé dans l'estomac,
ii) reproductibilité/répétabilité de la formation du radeau,
iii) certaines propriétés rhéologiques du radeau et notamment en termes de tenue (écoulement, consistance et viscosité du radeau).

En particulier, la composition selon l'invention permet la formation *in situ* d'un radeau gastrique gélifié de volume plus important et qui reste stable durant au moins 4 heures (par exemple durant au moins 6 heures), ce qui permet notamment de soulager un patient durant une grande partie de la nuit - voire durant la nuit entière - après l'administration de ladite composition au coucher.

Qui plus est, les inventeurs ont découvert, contre toute attente, que l'ajout d'une quantité efficace d'une préparation à base d'écorce d'orme rouge *(Ulmus rubra Muhl.)* (et en particulier à base d'écorce interne d'orme rouge) dans une préparation (par exemple pharmaceutique ou nutritionnelle) sous forme liquide ou solide à base d'acide alginique ou d'alginate, d'au moins un composé produisant un dégagement de dioxyde de carbone en milieu acide et éventuellement d'au moins un agent de réticulation de l'acide alginique (préparation pharmaceutique de type GAVISCON^{®}) permettait également d'obtenir de très bons résultats en matière de muco-adhésivité dudit radeau gastrique gélifié (pouvant également être qualifié de « gel moussant ») et d'induire de surcroît un effet anti-inflammatoire et/ou anti-oxydant. Ces très bons résultats en matière de muco-adhésivité sont liés au fait que le susdit radeau gastrique gélifié/gel moussant est capable d'adhérer, au moins partiellement, aux muqueuses au niveau local, par effet connexe de muco-adhésion. Sans être lié par la théorie, il est probable qu'un équilibre s'instaure au niveau local, notamment à la jonction gastro-œsophagienne, sur les muqueuses irritées, par muco-adhésion, du fait du délitement naturel du radeau dans l'organisme. La composition selon l'invention est donc adaptée pour former *in situ* une barrière protectrice « flottant » sur le contenu de l'estomac mais également présente sur la paroi oesophagienne pour apaiser la douleur et l'inflammation locale, notamment à la jonction gastro-œsophagienne. En cas de remontée gastrique acide, le radeau gastrique gélifié lui-même peut être régurgité dans l'œsophage et la couche/barrière protectrice muco-adhésive ainsi déposée sur la paroi œsophagienne va s'interposer entre la paroi œsophagienne et le liquide gastrique irritant, exerçant un effet protecteur et apaisant.

De surcroît, grâce à son pH proche de la neutralité et à sa fluidité et à sa flottabilité, le radeau gastrique gélifié obtenu après ingestion de la composition selon l'invention protège l'œsophage de l'acidité gastrique.

La préparation à base d'écorce d'orme rouge utilisée aux fins de la présente invention est une préparation à base de drogues végétales (plus simplement dénommée « préparation à base de plantes » ; la législation communautaire européenne privilégiant cette dénomination). Les préparations à base de drogues végétales sont des produits homogènes obtenus en soumettant les drogues végétales à des traitements tels que le broyage ou la coupe, l'extraction, la distillation, l'expression, le fractionnement, la purification, la concentration ou la fermentation. Ce sont, par exemple, des extraits, des huiles essentielles, des jus d'expression, des exsudats ayant subi un traitement, ou des drogues végétales ayant subi une opération de réduction de taille pour des applications spécifiques (par exemple, divisées pour des tisanes ou pulvérisées pour une encapsulation).

Avantageusement, l'on utilise, aux fins de la présente invention, une préparation à base d'écorce interne d'orme rouge (« slippery elm inner bark », en langue anglaise).

Selon un mode de réalisation de l'invention, la préparation à base d'écorce d'orme rouge est sélectionnée parmi une poudre d'écorce d'orme rouge, un extrait aqueux d'écorce d'orme rouge, un extrait hydro-alcoolique d'écorce d'orme rouge et/ou un extrait hydroglycériné d'écorce d'orme rouge.

La poudre d'écorce d'orme rouge correspond à la matière brute, à savoir à l'écorce d'orme rouge broyée grossièrement ou plus finement. De préférence, la granulométrie de ladite poudre est inférieure ou égale à 500µm (de préférence inférieure 500µm), préférablement inférieure ou égale à 400µm, avantageusement inférieure ou égale à 300µm, et se situe préférentiellement entre 5 et 250µm.

Concernant la préparation des extraits d'écorce d'orme rouge susvisés, il convient de noter que l'écorce de l'orme rouge est extraite avec de l'eau ou avec un mélange solvant eau / éthanol ou avec un mélange eau / glycérol, pour produire un extrait visqueux. L'extraction de l'écorce par exemple fragmentée ou pulvérisée, peut se faire par exemple par infusion à froid ou à chaud, par exemple par macération 15 minutes dans l'eau bouillante. L'extrait peut être réduit (concentré), lyophilisé ou séché par pulvérisation, mélangé ou non à un support de type maltodextrine.

Un extrait aqueux d'écorce interne d'orme rouge, *Ulmus rubra* Muhl, est obtenu, par exemple, par macération de la poudre obtenue en i) dans un volume d'eau purifiée suivant un rapport plante / extrait 1 /50, préférentiellement entre 1/5 et 1/10. L'extraction peut être réalisée dans une cuve inox ou autre matériau inerte, au moyen d'une agitation mécanique, préférentiellement avec un système à ultra-sons, ou micro-ondes. L'extraction peut être réalisée par livixation et /ou percolation du solvant sur la poudre. L'extrait ainsi obtenu est filtré, centrifugé. Il peut être concentré par évaporation sous vide et/ou lyophilisé.

Selon un mode de réalisation préféré, la préparation à base d'écorce interne d'orme rouge est sélectionnée parmi une poudre d'écorce interne d'orme rouge, un extrait aqueux d'écorce interne d'orme rouge et/ou un extrait hydroglycériné d'écorce interne d'orme rouge.

Selon un mode de réalisation particulièrement préféré, la préparation à base d'écorce interne d'orme rouge consiste en une poudre d'écorce interne d'orme rouge, avantageusement débarrassée des fibres dénommées « cotton cut » (pouvant être traduit en langue française par « coton »), le « cotton cut » étant un sous-produit obtenu lors du broyage de l'écorce interne d'orme rouge (défini, en langue anglaise, comme « a tan or whitish- to yellowish-brown matted mass of small fibers, punctuated with thin silvers of outer bark ; fibrous and pliable » ; que l'on peut traduire en langue française par « un amas de petites fibres, jaune foncé ou brun blanchâtre à brun jaunâtre, parsemé de petits éclats d'écorce externe ; fibreux et souple »).

### Définitions

**Acide alginique** / **alginate** : l'acide alginique (n° CAS : 9005-32-7) et ses dérivés (base conjuguée, sels et esters) les alginates sont des polysaccharides obtenus à partir d'une famille d'algues brunes : les laminaires ou les fucus.

Comme cela est connu de l'homme du métier, l'acide alginique est un polymère formé de deux monomères liés ensemble : le mannuronate (M) ou acide mannuronique dont certains sont acétylés et le guluronate (G) ou acide guluronique. La liaison se fait via β-1-4.

**Alginate de sodium** : l'alginate de sodium (n° CAS : 9005-38-3) est un sel extrait à partir de ce liquide visqueux issu de la paroi cellulaire d'algues brunes. Sa fonction naturelle consiste à augmenter la flexibilité de l'algue.

**Reflux gastro-œsophagien** *:* passage dans l'œsophage du contenu gastrique acide, qui se manifeste cliniquement par des brûlures rétrosternales ascendantes et des régurgitations acides.

**Pyrosis** (EN pyrosis, heartburn) : sensation de brûlure, à hauteur de l'estomac et de l'œsophage, qui peut s'accompagner de l'éructation d'un liquide acide. Elle traduit un reflux gastro-œsophagien.

**Gastrite** (EN gastritis) : Lésion inflammatoire de la muqueuse gastrique. Les gastrites peuvent se classer en gastrites aigües, chroniques et granulomateuses.

**Dyspepsie** (EN dyspepsia) : inconfort ou douleur récidivante ou persistante centrée après la prise alimentaire sur la partie haute de l'abdomen.

**Ulcère** (EN ulcer) : Perte de substance spontanée ne tendant pas à la cicatrisation normale.

**Ulcère gastroduodénal (UGD)** (EN peptic ulcer) : l'ulcère gastroduodénal résulte d'un déséquilibre entre l'agression chlorhydropeptique et les mécanismes de défense de la barrière muqueuse.

### Brève description des dessins

L'invention, sa fonctionnalité, ses applications ainsi que ses avantages seront mieux appréhendés à la lecture de la présente description, faite en référence aux figures 1 à 17, dans lesquelles :
- Les figures 1A et 1B sont des photographies permettant d'observer l'aspect du radeau de structure gélatineuse obtenu à partir du comprimé de l'exemple 2 dans le cadre de l'essai 1 de l'exemple 6, respectivement après 120 minutes à 37° et après 20 heures à 37°,
- Les figures 2A et 2B sont des photographies permettant d'observer l'aspect du radeau obtenu à partir du produit de référence dans le cadre de l'essai 1 de l'exemple 6, respectivement après 30 minutes à 37° et après 20 heures à 37°,
- La figure 3 est une photographie montrant le radeau obtenu à partir du comprimé de l'exemple 2, formé après refroidissement d'une heure en mettant en oeuvre le mode opératoire de l'essai 2 de l'exemple 6,
- La figure 4 est une photographie permettant d'observer le radeau obtenu à partir du comprimé de l'exemple 2 en mettant en oeuvre le mode opératoire de l'essai 2 de l'exemple 6 sur le dispositif de filtration, après 45 minutes d'égouttage,
- La figure 5 est une photographie montrant le radeau obtenu à partir du produit de référence formé après seulement 30 minutes d'incubation à 37° en mettant en œuvre le mode opératoire de l'essai 2 de l'exemple 6,
- La figure 6 est une photographie montrant le radeau obtenu à partir du produit de référence, formé après refroidissement d'une heure en mettant en oeuvre le mode opératoire de l'essai 2 de l'exemple 6,
- Les figures 7 à 16 sont des images obtenues en microscopie électronique illustrant les résultats obtenus à l'exemple 7,
- La figure 17 est un schéma illustrant les différentes étapes du procédé de préparation de la poudre d'écorce interne d'orme rouge.

### Description détaillée

Les exemples ci-après permettront de mieux appréhender la présente invention. Toutefois, ces exemples ne sont donnés qu'à titre illustratif et ne doivent en aucun cas être regardés comme limitant la portée de ladite invention d'une quelconque manière.

### Exemples

### Exemple 1 : Procédé de préparation de la poudre d'écorce interne d'orme rouge utilisée dans les compositions des exemples 2-5

La poudre d'écorce interne d'orme rouge, *Ulmus rubra* Muhl, provient des Etats-Unis d'Amérique (USA). La couche externe de l'écorce est enlevée et rejetée, exposant l'écorce interne souhaitée. Après enlèvement de l'écorce extérieure, l'écorce interne peut être détachée (par exemple en bandes, carrés ou copeaux). L'écorce interne est ensuite pulvérisée par un procédé de cryobroyage avec de l'azote liquide à une température comprise entre -2,0°C et -160°C, préférentiellement à une température comprise entre -60 et -120° C, de manière à obtenir une poudre dont la granulométrie est inférieure à 500µm et se situe préférentiellement entre 5 et 250µm.

A noter que l'étape de broyage peut également être mise en œuvre de manière plus traditionnelle, à savoir au moyen d'un broyeur ou d'un broyeur à marteaux.

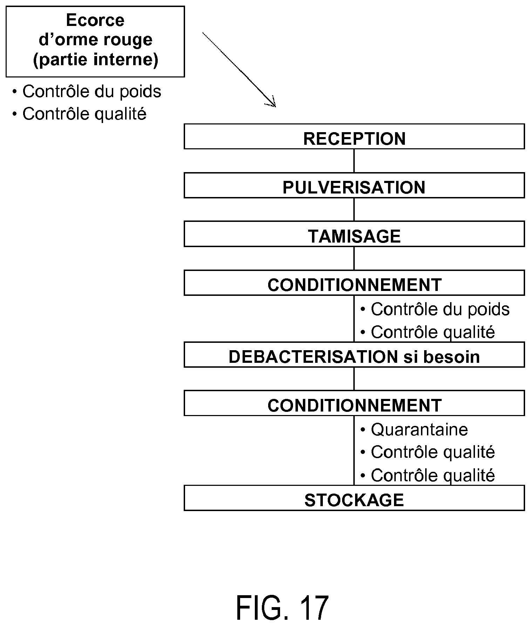

Plus précisément les différentes étapes du procédé de préparation de la susdite poudre d'écorce interne d'orme rouge sont schématisées en figure 17.

En mettant en œuvre ce procédé, l'on obtient une poudre d'écorce interne d'orme rouge possédant les caractéristiques suivantes (cf. tableau 1 infra) :

**Tableau 1**

| | |
|---|---|
| **Origine** | USA |
| **Partie de la plante utilisée** | Ecorce |

| **CARACTERISTIQUES ORGANOLEPTIQUES** | |
|---|---|
| Apparence | Poudre fine |
| Couleur | Beige rosé |
| Odeur | Caractéristique |

| **CARACTERISTIQUES PHYSICO-CHIMIQUES** | |
|---|---|
| Perte à la dessiccation | ≤ 10.0% |
| Granulométrie | ≤ 250 µm |
| | Tolérance 100%≤ 500 µm |

| **CONTAMINANTS** | |
|---|---|
| Pesticides | Conforme à la Ph. Eur. et à la règlementation européenne en vigueur |
| Plomb | ≤ 3.0 ppm |
| Cadmium | ≤ 1.0 ppm |
| Mercure | ≤ 0.1 ppm |
| Aflatoxines | Aflatoxine B1 ≤ 2.0 ppb Aflatoxines B1, B2, G1, G2 ≤ 4.0 ppb |
| 4HAP | Benzo(a)pyrène ≤ 10.0 ppb 4HAP ≤ 50.0 ppb |

| **CARACTERISTIQUES MICROBIOLOGIQUES (Ph. Eur. Édition en vigueur)** | |
|---|---|
| Germes aérobies totaux (DGAT) ≤ 10 000 UFC/g | |
| Moisissures et levures totales (DMLT) ≤ 100 UFC/g | |
| Bactéries gram - / Entérobactéries ≤ 100 UFC/g | |
| *E. coli* Absent /g | |
| *Staphylococcus aureus* Absent /g | |
| *Salmonelles* Absent /10g | |

| **REGLEMENT ATION** | |
|---|---|
| Matière non OGM (Règlements CE 1829/2003 et 1830/2003 et leurs modifications) | |
| Matière non soumise à l'étiquetage Allergènes (Directives 2000/13/CE et ses modifications) | |

| **STOCKAGE ET DUREE DE VIE** | |
|---|---|
| Stocker dans l'emballage d'origine hermétiquement fermé à température ambiante | |
| Durée de vie : 3 ans | |

### Exemple 2 : Procédé de préparation d'une composition selon l'invention (forme solide ; comprimé à croquer)

Le présent exemple vise à illustrer un procédé de préparation d'une composition selon l'invention, sous forme d'un comprimé à croquer de 1700 mg, dont la composition est présentée dans le tableau 2 infra :

**Tableau 2**

| **Pourcentage massique (% m)** | **Ingrédients** | **mg** / **comprimé** |
|---|---|---|
| 15.71 | Bicarbonate de sodium | 267 |
| 14.71 | Poudre d'orme rouge (écorce interne) | 250 |
| 9.41 | Carbonate de calcium | 160 |
| 9.76 | Alginate de sodium | 166 |
| 2.00 | Arôme poudre de menthe spearmint | 34 |
| 45.42 | Sorbitol | 772,1 |
| 0.19 | Stevia | 3,3 |
| 2.00 | Mono-, di- et triesters d'acide béhénique (E471) | 34 |
| 0.80 | Stéarate de magnésium | 13,6 |

Dans la composition objet du présent exemple - ainsi que dans celle décrite dans les exemples 2-4 infra - l'on utilise un alginate de sodium commercialisé par la société KIMICA Corporation sous la dénomination « KIMICA ALGIN IL-6G ». Cet alginate de sodium présente les propriétés suivantes :
- une viscosité :
   i) comprise entre 35 et 65 centipoises dans une solution aqueuse à 1% (poids/volume), en utilisant un viscosimètre de Brookfield (spindle #1) à une vitesse de rotation de 30 t/min (30 rpm) et à une température de 20°C; et
   ii) comprise entre 7000 et 14000 centipoises dans une solution aqueuse à 6% (poids/volume), en utilisant un viscosimètre de Brookfield (spindle #3) à une vitesse de rotation de 3 t/min (3 rpm) et à une température de 20°C ;
- un pH en solution aqueuse à 1 % (poids/volume) compris entre 6 et 8,
- une taille de particules définie comme suit : 95% min à travers une maille de « 35 Mesh/425µm (« 95% min through 35 Mesh/ 425µm », en langue anglaise), et
- résistance du gel ("Gel strength", en langue anglaise ): 50g min.

La composition sous forme de comprimé à coquer présentée dans le tableau 2 supra est obtenue en mettant en oeuvre les étapes 1-5 résumées ci-après :

### 1 - Vide ligne :

Pesée des ingrédients

### 2 - Mélange :

a-Pré-mélange de sucre et de stevia dans un mélangeur cubique, durant un temps défini.
b-Mélange des ingrédients dans un mélangeur à vis hélicoïdale ou un mélangeur pneumatique, après tamisage :
   Sorbitol, bicarbonate de sodium, sucre, pré-mélange de sucre et de stevia, mono et diglycérides d'acides gras, carbonate de calcium, stéarate de magnésium, fructose, poudre d'écorce d'orme rouge, alginate de sodium, arome de menthe en poudre.

Le mélange est réalisé selon un temps défini avec le matériel utilisé.

Le contenu du mélangeur est vidé dans des sacs doubles en PEBD (ou équivalent) de qualité adaptée au contact alimentaire.

### 3 - Compression :

La compression est réalisée à l'aide d'une presse à comprimer rotative équipée avec des poinçons adaptés.

### 4- Conditionnement primaire et secondaire :

Conditionnement primaire dans des blisters, selon les besoins les blisters sont conditionnés dans des saches en aluminium qui sont ensuite fermées de manière étanche.

Les blisters ou les saches sont conditionnés dans des boîtes de carton avec insertion d'une notice par boîte. Sur la boîte figurent les mentions obligatoires et adaptées du produit.

### 5 - Contrôle du produit fini :

Contrôle du produit fini par le laboratoire de contrôle de la qualité et libération par la personne responsable de la libération du produit.

### Exemple 3 : Composition selon l'invention (également sous forme solide [comprimé à croquer] mais différente de celle objet de l'exemple 2)

Le procédé décrit à l'exemple 2 est mis en œuvre et adapté afin d'obtenir un comprimé à croquer dont la composition est présentée dans le tableau 3 infra :

**Tableau 3**

| **Pourcentage massique (% m)** | **Ingrédients** | **mg / comprimé** |
|---|---|---|
| 13.35 | Bicarbonate de sodium | 267 |
| 10 | Poudre d'orme rouge (écorce interne) | 200 |
| 8 | Carbonate de calcium | 160 |
| 8.3 | Alginate de sodium | 166 |
| 4 | Arôme poudre de menthe spearmint | 80 |
| 2.25 | Fructose | 45 |
| 1.75 | Sucre surfin | 35 |
| 49.845 | Sorbitol | 996.9 |
| 0.005 | Stevia | 0.1 |
| 1.7 | Mono-, di- et triesters d'acide béhénique (E471) | 34 |
| 0.8 | Stéarate de magnésium | 16 |

### Exemple 4 : Composition selon l'invention (également sous forme solide [comprimé à croquer] mais différente de celle objet des exemples 2 et 3)

Le procédé décrit à l'exemple 2 est mis en œuvre et adapté afin d'obtenir un comprimé à croquer dont la composition est présentée dans le tableau 4 infra :

**Tableau 4**

| **Pourcentage massique (% m)** | **Ingrédients** | **mg / comprimé** |
|---|---|---|
| 13.35 | Bicarbonate de sodium | 267 |
| 10 | Poudre d'orme rouge (écorce interne) | 200 |
| 8 | Carbonate de calcium | 160 |
| 8.3 | Alginate de sodium | 166 |
| 4 | Arôme poudre de menthe spearmint | 80 |
| 2.25 | Fructose | 45 |
| 1.75 | Sucre surfin | 35 |
| 49.845 | Isomalt | 996.9 |
| 0.005 | Stevia | 0.1 |
| 1.7 | Mono-, di- et triesters d'acide béhénique (E471 ) | 34 |
| 0.8 | Stéarate de magnésium | 16 |

### Exemple 5 : Procédé de préparation d'une composition selon l'invention (sous forme liquide)

Le présent exemple vise à illustrer un procédé de préparation d'une composition selon l'invention, sous forme liquide ; par exemple sous forme d'un liquide visqueux/gel présenté en stick (5 ou 10 ml) ou sous forme sirop. Cette composition est présentée dans le tableau 5 infra :

**Tableau 5**

| **Matières Premières** | **Masse unitaire (masse par unité de prise) mg** | **Pourcentage massique par unité de prise** |
|---|---|---|
| Carbonate de calcium | 160 | |
| Bicarbonate de sodium | 267 | |
| Alginate de sodium | 490 | |
| Poudre d'orme rouge | 10 | |
| Arôme de menthe | | 0.40% |
| Glycérine | | 42.50% |
| Sorbitol | | 30% |
| Sirop de fructose | | 10% |
| Eau purifiée | | 8.70% |

Dans la composition objet du présent exemple, on utilise un alginate de sodium commercialisé par la société KIMICA Corporation sous la dénomination « KIMICA ALGIN ULV-5G ». Cet alginate de sodium présente les propriétés suivantes :
- une viscosité comprise entre 300 et 700 centipoises dans une solution aqueuse à 10% (poids/volume) et à une température de 20°C,
- un pH en solution aqueuse à 1 % (poids/volume) compris entre 6 et 8, et
- une taille de particules définie comme suit : 95% min à travers une maille de « 80 Mesh/180µm (« 95% min through 80 Mesh/180µm», en langue anglaise).

La composition sous forme liquide présentée dans le tableau 5 supra (présentée en stick ou sous forme sirop) est obtenue en mettant en œuvre les étapes 0 à 4 suivantes :
*0- Vide de ligne*
*1- Pesée des matières premières*
*2- Mélange:*
   Pré-mélange aromatique
   puis Mélange de tous les ingrédients, dans un mélangeur, à température définie et contrôlée, avec dégazage, et avec ou sans décontamination microbiologique.
*3- Conditionnement primaire et secondaire, avec ou sans décontamination microbiologique,*
*4- Contrôle final et libération.*

### Exemple 6 : Comparaison des propriétés physico-chimiques et rhéoloqiques du radeau gélifié obtenu à partir du comprimé de l'exemple 2 et de celui obtenu à partir d'un produit de référence (GAVISCON^{®} MENTHE, comprimé à croquer 1700 mg)

### 6.1. Introduction / objectifs

Le présent exemple vise à comparer les propriétés physico-chimiques et rhéologiques des radeaux gastriques obtenus à partir de la composition selon l'invention (comprimé obtenu à l'exemple 2) et le produit de référence, et ce dans des conditions spécifiques à chaque essai.

Chaque essai a été répété sur n=10 prises d'essai afin d'évaluer non seulement la performance de l'essai mais aussi sa répétabilité, propriété particulièrement importante.

### 6.2. Matériel et méthodes

### 6.2.a. Produits analysés

Les produits analysés sont les suivants :

| | **Désignation** | **Lot** | **Titulaire/Fabricant** |
|---|---|---|---|
| **1. Comprimé de l'exemple 2** | Comprimé de l'exemple 2 | 280416-01 (lot pilote) | ARKOPHARMA |
| | Comprimé à croquer de 1700 mg (16mm de diamètre) | | |

| | **Lots** | **Désignation** | **Titulaire/Fabricant** |
|---|---|---|---|
| 2. **Produit de référence** | 529204/534820/602219 604104 | GAVISCON^{®} MENTHE, comprimé à croquer 1700 mg | RECKITT BENCKISER HEALTHCARE (UK) |
| | **Composition** | | |
| | GAVISCON^{®} menthe, comprimé à croquer Liste complète des substances actives et des excipients pour un comprimé à 1700 mg | | |
| | *Substances actives* | | |
| | Alginate de sodium | | 500,00 mg |
| | Bicarbonate de sodium | | 267,00 mg |
| | Carbonate de calcium | | 160,00 mg |
| | *Autres composants* | | |
| | Arôme menthe, macrogol 20 000, mannitol (E421), aspartam (E951), stéarate de magnésium, copovidone, acésulfame potassique (E950). | | |

### 6.2.b. Mesures réalisées

Les essais comparatifs suivants ont été effectués sur les produits à analyser présentés au point 6.2.a. supra :
- Essai 1 : Suivi de la formation et de la stabilité du radeau gastrique dans le temps
- Essai 2 : Poids du radeau gastrique

Essais rhéologiques (essais 3-5) :
- Essai 3 : Durée d'écoulement (coupe d'écoulement ISO)
- Essai 4 : Mesure de la consistance du gel (consistomètre de Bostwick - Endecotts Ltd. Royaume Uni Réf. : ZXCON-CON1, N° d'inventaire : 6089)
- Essai 5 : Viscosité du gel (viscosimètre de Brookfield)
- Essai 6 : Détermination du pH 22°C du gel

### 6.2.c. Appareillages spécifiques pour les essais

Plaque d'agitation à barreau aimanté et à vitesse étalonnée.

Un agitateur magnétique IKA C-MAG HS 7 en raison de sa capacité d'agitation et de sa plage d'agitation finement ajustable.

Avant les essais, les graduations de vitesses 3,4 et 5 de l'agitateur ont été étalonnées à l'aide d'un tachymètre en utilisant un barreau de taille identique.

**Tableau d'équivalence n° de vitesse/rpm**

| **Graduations de vitesse** | **Rotation par minute [ rpm] mesurée (n=3)** |
|---|---|
| 3 | (790+802+797)/3 = 796 |
| 4 | (1080+1065+1067)/3 = 1071 |
| 5 | (1400+1406+1394)/3 = 1400 |

### Barreau aimanté

Nous avons utilisé pour tous les essais un barreau aimanté triangulaire enrobé de PTFE de longueur 35 mm et dont la section est un triangle équilatéral de 9 mm. Préconisée pour la dissolution des solides ou le mélange de sédiments ou de poudre, la forme triangulaire permet une action de raclage sur le fond du bécher et permet une formation du gel rapide et homogène.

### Agitateur secoueur orbital

Il s'agit d'un agitateur à plateau à mouvement orbital de type IKA Vibrax-VXR.

### Dispositif de filtration

Il s'agit d'un tissu^{*} (carré 200 mm × 200 mm) en polyester (PES 100%) d'une maille de 1 mm. Ce tissu est maintenu de manière tendue sur un entonnoir Nalgene, de diamètre 10 cm à l'aide d'élastiques. Ce dispositif de filtration est taré puis posé sur un bécher qui de support et de collecteur du filtrat.

**tissu Moustiquaire INSECT PROTECT* - *Windhager France S.A.R.L. Art. Nr. 03789 N0217* ERICHSEN ISO 2431 Nr.6 04/331

### 6.3. Essai 1 (suivi de la formation et de la stabilité du radeau gélifié dans le temps)

### 6.3.a. Mode opératoire

Broyer un comprimé de 1700 mg dans un mortier en porcelaine jusqu'à obtention d'une poudre fine et homogène. Transférer la poudre de comprimé quantitativement dans un bécher 150 ml de forme haute. Pour la dispersion de la poudre, mettre sous agitation à la vitesse 3 puis ajouter 50 ml d'acide chlorhydrique à 0,1N/37°C. Laisser sous agitation magnétique à la température ambiante du laboratoire (22°C) pendant exactement 20 secondes à la vitesse 3. Augmenter la vitesse d'agitation à 4 pendant exactement 20 secondes puis rebaisser la vitesse à 3 et continuer l'agitation pendant exactement 10 secondes. Transférer rapidement en un seul coup la totalité (gel en formation et phase liquide acide) dans une éprouvette graduée, d'un volume de 100 ml. Incuber le tout dans un bain marie à 37,0°C pendant 120 minutes. Noter le volume et le positionnement du radeau dans l'intervalle de temps suivant. T0, T30, T60 et T120 minutes.

Remarque : Nous avons laissé incuber les essais au-delà du temps T120 pendant la nuit pour évaluer l'évolution du radeau à long terme à 37°C.

### 6.3.b. Observations concernant l'essai 1

### 6.3.b.1 Concernant le comprimé de l'exemple 2

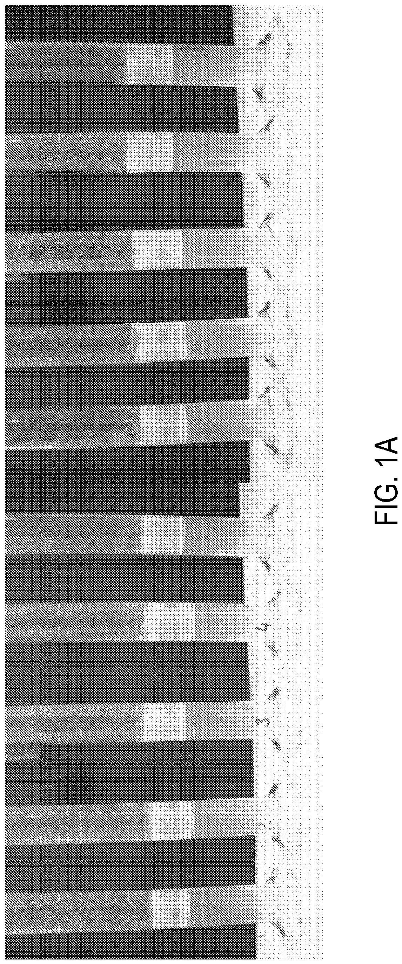

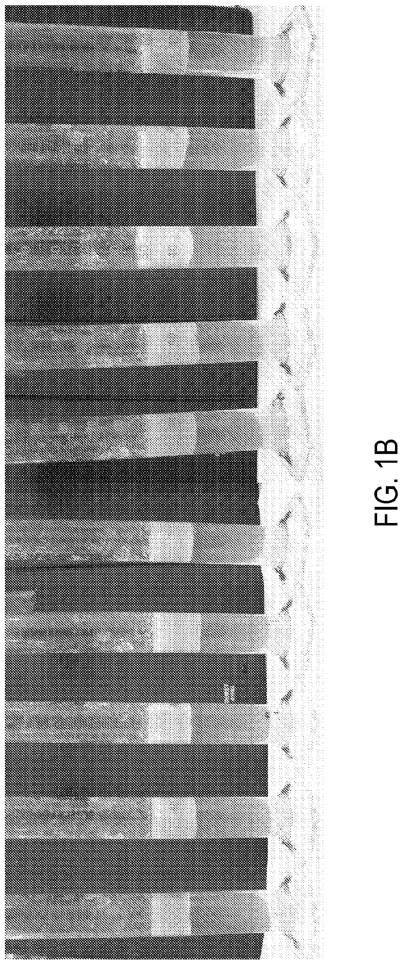

Après 120 minutes à 37°C, le radeau de structure gélatineuse est parfaitement stable et très dense (cf. figure 1A). Après 20 heures à 37°C, l'aspect du radeau reste inchangé puis nous observons un léger trouble au fond de l'éprouvette graduée (cf. figure 1B).

### 6.3.b.2 Concernant le produit de référence

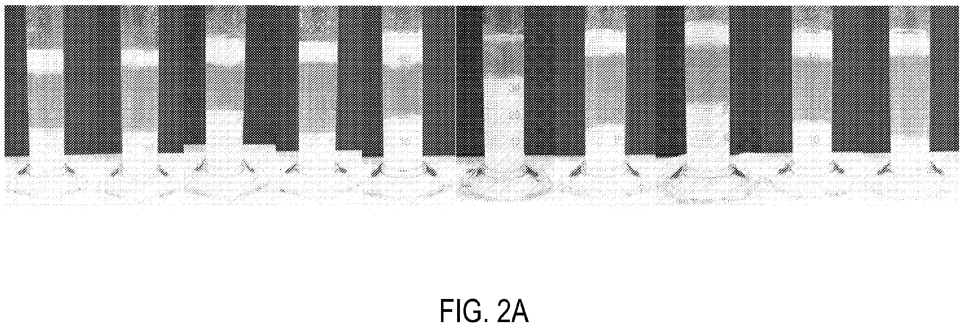

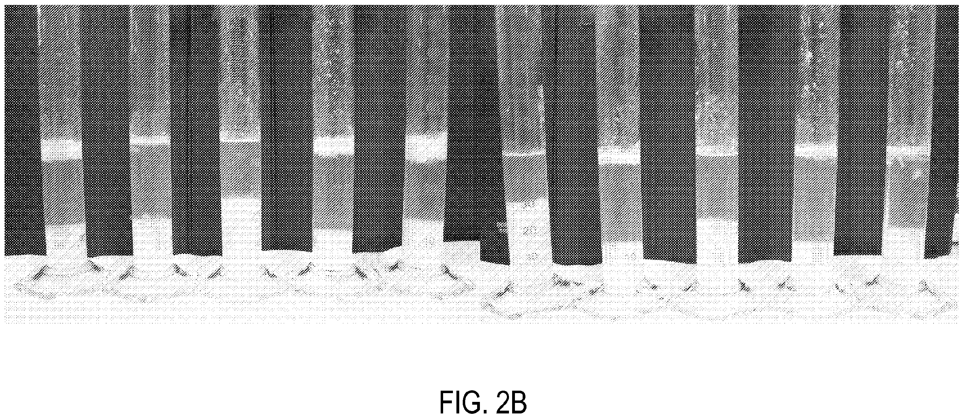

Comme en témoigne la figure 2A, après seulement 30 minutes à 37°C, le radeau est déjà plus ou moins dégradé, peu dense et la structure ne ressemble pas à un gel. Au fond de l'éprouvette graduée, la partie « plongée » du radeau (partie du radeau ayant plongé/coulé au fond de l'éprouvette) est bien visible. Il s'agit d'une matière floconneuse en dispersion. Le radeau est complètement instable et friable depuis le début de l'essai. Après 20 heures d'essai, il ne s'agit plus d'un radeau sur la ligne de flottaison à 50 ml mais de particules accrochées sur les parois du verre (cf. figure 2B).

### 6.3.c. Résultats de l'essai 1

### 6.3.c.1. Concernant le comprimé de l'exemple 2

Les résultats de l'essai 1 concernant le comprimé de l'exemple 2 sont récapitulés dans le tableau 6 infra.

**Tableau 6**

| **1. COMPRIME DE L'EXEMPLE 2** | | |
|---|---|---|
| temps [min] | volume [ml] du radeau | position du radeau ml_min à ml_max |
| 0 | | |
| 30 | 14 | 37-51 fond 0 |
| 60 | 14 | 37-51 fond 0 |
| 120 | 14 | 37-51 fond 0 |
| 20h37 | 14 | 37-51 fond < 1 |

| **2. COMPRIME DE L'EXEMPLE 2** | | |
|---|---|---|
| temps [min] | volume [ml] du radeau | position du radeau ml_min à ml_max |
| 0 | | |
| 30 | 15 | 35-50 fond 0 |
| 60 | 15 | 35-50 fond 0 |
| 120 | 15 | 35-50 fond 0 |
| 20h37 | 15 | 35-50 fond < 1 |

| **3. COMPRIME DE L'EXEMPLE 2** | | |
|---|---|---|
| temps [min] | volume [ml] du radeau | position du radeau ml_min à ml_max |
| 0 | | |
| 30 | 15 | 36-51 fond 0 |
| 60 | 16 | 36-52 fond 0 |
| 120 | 16 | 36-52 fond 0 |
| 20h37 | 16 | 36-52 fond < 1 |

| **4. COMPRIME DE L'EXEMPLE 2** | | |
|---|---|---|
| temps [min] | volume [ml] du radeau | position du radeau ml_min à ml_max |
| 0 | | |
| 30 | 17 | 33-50 fond 0 |
| 60 | 17 | 33-50 fond 0 |
| 120 | 17 | 33-50 fond 0 |
| 20h37 | 17 | 33-50 fond < 1 |

| **5. COMPRIME DE L'EXEMPLE 2** | | |
|---|---|---|
| temps [min] | volume [ml] du radeau | position du radeau ml_min à ml_max |
| 0 | | |
| 30 | 16 | 35-51 fond 0 |
| 60 | 16 | 35-51 fond 0 |
| 120 | 16 | 35-51 fond 0 |
| 20h37 | 14 | 37-51 fond < 1 |

| **6. COMPRIME DE L'EXEMPLE 2** | | |
|---|---|---|
| temps [min] | volume [ml] du radeau | position du radeau ml_min à ml_max |
| 0 | | |
| 30 | 17 | 34-51 fond 0 |
| 60 | 18 | 34-52 fond 0 |
| 120 | 18 | 34-52 fond 0 |
| 20h37 | 16 | 35-51 fond < 1 |

| **7. COMPRIME DE L'EXEMPLE 2** | | |
|---|---|---|
| temps [min] | volume [ml] du radeau | position du radeau ml_min à ml_max |
| 0 | | |
| 30 | 17 | 34-51 fond 0 |
| 60 | 18 | 33-51 fond 0 |
| 120 | 18 | 33-51 fond 0 |
| 20h37 | 17 | 33-50 fond < 1 |

| **8. COMPRIME DE L'EXEMPLE 2** | | |
|---|---|---|
| temps [min] | volume [ml] du radeau | position du radeau ml_min à ml_max |
| 0 | | |
| 30 | 17 | 33-50 fond 0 |
| 60 | 17 | 34-51 fond 0 |
| 120 | 17 | 34-51 fond 0 |
| 20h37 | 17 | 37-54 fond < 1 |

| **9. COMPRIME DE L'EXEMPLE 2** | | |
|---|---|---|
| temps [min] | volume [ml] du radeau | position du radeau ml_min à ml_max |
| 0 | | |
| 30 | 15 | 37-52 fond 0 |
| 60 | 15 | 37-52 fond 0 |
| 120 | 15 | 37-52 fond 0 |
| 20h37 | 15 | 37-52 fond < 1 |

| **10. COMPRIME DE L'EXEMPLE 2** | | |
|---|---|---|
| temps [min] | volume [ml] du radeau | position du radeau ml_min à ml_max |
| 0 | | |
| 30 | 16 | 37-53 |
| 60 | 16 | 37-53 |
| 120 | 16 | 37-53 |
| 20h37 | 16 | 37-53 fond < 1 |
| | | |
| **moyenne à 30 min. n = 10** | 16 | |
| **écart-type** | 1 | |
| **%RSD** | 8.3 | |

Tel qu'indiqué dans le tableau 6 supra, le volume moyen du radeau de 16 ml après 30 minutes est très répétable (coefficient de variation de 8,3% sur 10 essais avec un min de 14 ml et un max de 17 ml) et reste compact au-delà de la durée d'observation à 37°C. La position, l'espace de flottaison du radeau reste pendant tout ce temps sensiblement identique et la formation d'un fond reste quantitativement négligeable.

### 6.3.c.2. Concernant le produit de référence

Les résultats de l'essai 1 concernant le produit de référence sont récapitulés dans le tableau 7 infra.

**Tableau 7**

| **1. PRODUIT DE REFERENCE** | | |
|---|---|---|
| temps [min] | volume [ml] du radeau | position du radeau ml_min à ml_max |
| 0 | | |
| 30 | 10 | 41-51 fond 17 |
| 60 | 9 | 43-52 fond 17 |
| 180 | 8 | 43-51 fond 17 |
| 290 | 8 | 43-51 fond 17 |
| 20h52min | 7 | 44-51 fond 17 |

| **2. PRODUIT DE REFERENCE** | | |
|---|---|---|
| temps [min] | volume [ml] du radeau | position du radeau ml_min à ml_max |
| 0 | | |
| 30 | 7 | 45-52 fond 18 |
| 60 | 6 | 46-52 fond 17 |
| 180 | 5 | 46-51 fond 16 |
| 290 | 5 | 46-51 fond 16 |
| 20h52min | 1 | 50-51 fond 20 |

| **3. PRODUIT DE REFERENCE** | | |
|---|---|---|
| temps [min] | volume [ml] du radeau | position du radeau ml_min à ml_max |
| 0 | | |
| 30 | 10 | 41-51 fond 24 |
| 60 | 9 | 42-51 fond 23 |
| 180 | 1 | 50-51 fond 30 |
| 290 | 1 | 50-51 fond 30 |
| 20h52min | 1 | 50-51 fond 28 |

| **4. PRODUIT DE REFERENCE** | | |
|---|---|---|
| temps [min] | volume [ml] du radeau | position du radeau ml_min à ml_max |
| 0 | | |
| 30 | 8 | 43-51 fond 15 |
| 60 | 6 | 45-51 fond 15 |
| 180 | 6 | 45-51 fond 15 |
| 290 | 6 | 45-51 fond 15 |
| 20h52min | 6 | 45-51 fond 15 |

| **5. PRODUIT DE REFERENCE** | | |
|---|---|---|
| temps [min] | volume [ml] du radeau | position du radeau ml_min à ml_max |
| 0 | | |
| 30 | 11 | 41-52 fond 20 |
| 60 | 11 | 41-52 fond 20 |
| 180 | 10 | 42-52 fond 19 |
| 290 | 10 | 42-52 fond 19 |
| 20h52min | 9 | 43-52 fond 19 |

| **6. PRODUIT DE REFERENCE** | | |
|---|---|---|
| temps [min] | volume [ml] du radeau | position du radeau ml_min à ml_max |
| 0 | | |
| 30 | 1 | 50-51 fond 34 |
| 60 | 1 | 50-51 fond 34 |
| 180 | 1 | 50-51 fond 33 |
| 290 | 1 | 50-51 fond 33 |
| 20h52min | 1 | 50-51 fond 31 |

| **7. PRODUIT DE REFERENCE** | | |
|---|---|---|
| temps [min] | volume [ml] du radeau | position du radeau ml_min à ml_max |
| 0 | | |
| 30 | 9 | 43-52 fond 15 |
| 60 | 7 | 45-52 fond 15 |
| 180 | 6 | 45-51 fond 15 |
| 290 | 5 | 46-51 fond 15 |
| 20h52min | 5 | 46-51 fond 16 |

| **8. PRODUIT DE REFERENCE** | | |
|---|---|---|
| temps [min] | volume [ml] du radeau | position du radeau ml_min à ml_max |
| 0 | | |
| 30 | 8 | 44-52 fond 24 |
| 60 | 8 | 44-52 fond 17 |
| 180 | 6 | 45-51 fond 22 |
| 290 | 1 | 50-51 fond 27 |
| 20h52min | 1 | 50-51 fond 25 |

| **9. PRODUIT DE REFERENCE** | | |
|---|---|---|
| temps [min] | volume [ml] du radeau | position du radeau ml_min à ml_max |
| 0 | | |
| 30 | 9 | 43-52 fond 13 |
| 60 | 7 | 44-51 fond 12 |
| 180 | 6 | 45-51 fond 17 |
| 290 | 6 | 45-51 fond 18 |
| 20h52min | 2 | 49-51 fond 15 |

| **10. PRODUIT DE REFERENCE** | | |
|---|---|---|
| temps [min] | volume [ml] du radeau | position du radeau ml_min à ml_max |
| 0 | | |
| 30 | 6 | 45-51 fond 12 |
| 60 | 6 | 45-51 fond 11 |
| 180 | 6 | 45-51 fond 12 |
| 290 | 5 | 46-51 fond 14 |
| 20h52min | 1 | 50-51 fond 20 |
| | | |
| **moyenne à 30 min. n = 10 écart-type %RSD** | 8 | |
| | 3 | |
| | 33.8 | |

Tel que mentionné dans le tableau 7 supra, le volume moyen du radeau restant après 30 minutes est de 8 ml et il ne ressemble en rien à un gel, mais plutôt à un agglomérat de matière à faible densité. Le volume de radeau restant n'est pas répétable (CV de 33,5 % avec un min de 1 ml et un max de 11 ml). Le volume de la matière au fond de l'éprouvette fait à lui tout seul souvent plus que 50 % du volume du radeau encore en état de flottaison. L'essai n°6 n'a plus de radeau au point de mesure T30 minutes. La position du radeau change et diminue rapidement dans le temps. Au même moment le fond de l'éprouvette se remplit avec une matière de structure amorphe.

### 6.3.d. Conclusion de l'essai 1

Les principaux enseignements de l'essai 1 sont récapitulés dans le tableau 8 infra.

**Tableau 8**

| | **Comprimé de l'exemple 2** | **Produit de référence (GAVISCON^{®})** |
|---|---|---|
| **Volume moyen à 30min (n=10)** | 16 ml | 8 ml |
| **Ecart-type** | 1 | 3 |
| **%RSD** | 8,3 | 33,8 |

De manière particulièrement significative, la présence de la poudre d'écorce interne d'orme rouge dans la composition selon l'invention permet de doubler le volume du radeau avec une reproductibilité de formation plus de quatre fois supérieure par rapport au produit de référence (GAVISCON^{®}).

### 6.4. Essai 2 (poids du radeau)

### 6.4.a. Mode opératoire

Après avoir confectionné préalablement à l'essai le dispositif de filtration, broyer un comprimé de 1700 mg dans un mortier en porcelaine jusqu'à obtention d'une poudre fine et homogène. Transférer la poudre de comprimé quantitativement dans un bécher 150 ml de forme haute. Pour disperser la poudre, mettre sous agitation à la vitesse 3 puis ajouter 50 ml d'acide chlorhydrique à 0,1N/37°C. Laisser sous agitation magnétique à la vitesse 3 à la température ambiante du laboratoire (22°C) pendant exactement 20 secondes. Augmenter la vitesse d'agitation à 4 pendant exactement 20 secondes puis rebaisser la vitesse à 3 et continuer l'agitation pendant exactement 10 secondes. Pour la formation du gel (radeau), laisser reposer en incubant pendant 30 minutes au bain marie à 37,0°C. Laisser revenir pendant 60 minutes à la température ambiante du laboratoire de 22°C. Exercer avec l'index une légère pression au centre du tamis dans le but de donner à la surface filtrante une forme concave pour éviter le débordement du gâteau de filtration (radeau). Maintenant, verser lentement, si possible la phase liquide d'abord puis la fraction gélifiée (radeau) en totalité dans le centre du dispositif de filtration qui a été préalablement taré. Pendant cette opération, le barreau magnétique d'agitation est maintenu au fond du bécher à l'aide d'un barreau externe. Laisser égoutter pendant exactement 60 minutes à la température du laboratoire (22°C) puis peser le dispositif de filtration avec le radeau en absence de gouttes de liquide sous le tamis et sur la surface de l'entonnoir. Si nécessaire, sécher la surface inférieure du dispositif de filtration avec un papier absorbant sans toucher le tamis. Il ne faut pas dessécher le radeau par effet de capillarité du papier.

### 6.4.b. Observations concernant l'essai 2

### 6.4.b.1. Concernant le comprimé de l'exemple 2

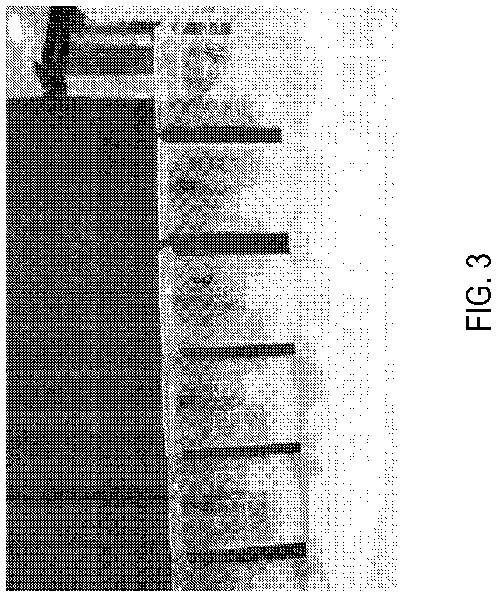

Le radeau formé est observé après refroidissement d'une heure. Comme illustré en figure 3, la phase supérieure flottante (radeau) est bien formée et reste formée. La phase inférieure est très peu trouble et de couleur jaunâtre. Nous n'observons pas de fond.

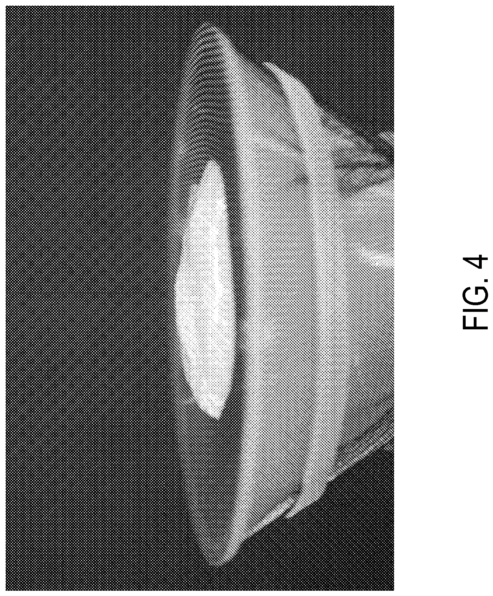

Le radeau est observé sur le dispositif de filtration, après 45 minutes d'égouttage. Le radeau est facilement séparable par le système de filtration de la phase liquide inférieure et le temps d'égouttage est bien choisi. Une vue de près du radeau sur le dispositif de filtration est montrée en figure 4.

### 6.4.b.2. Concernant le produit de référence

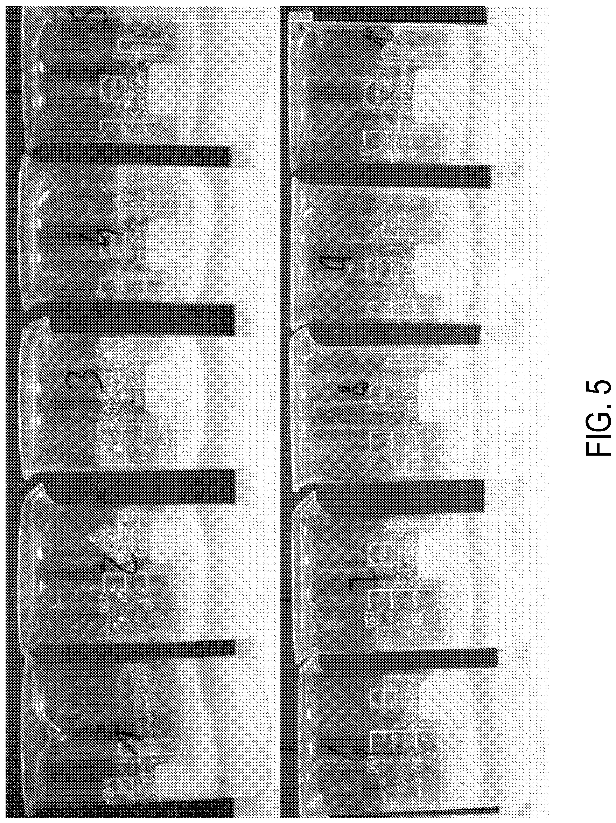

Le radeau formé après 30 minutes d'incubation à 37°C est observé. Comme montré en figure 5, le radeau après seulement 30 minutes d'incubation à 37°C est déjà en train de se dégrader. Une quantité non négligeable de matière est bien visible au fond du bécher et correspond au radeau ayant plongé/coulé.

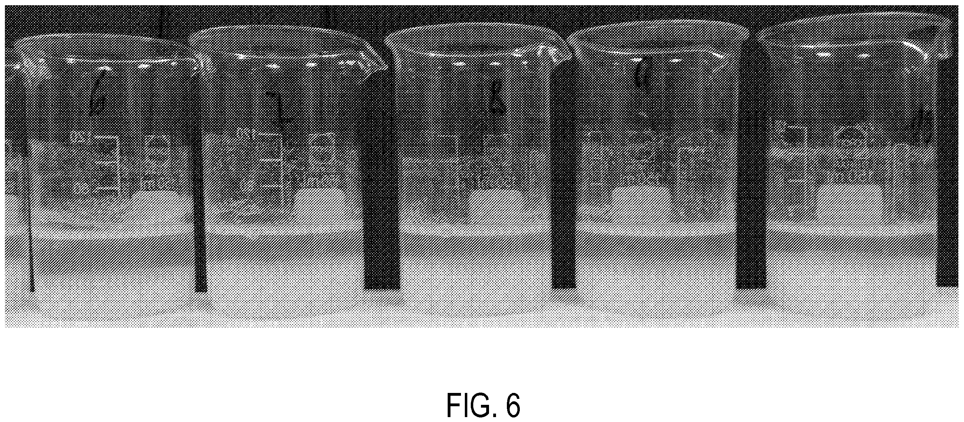

Après une heure de refroidissement, les radeaux n'existent plus (cf. figure 6) et les gâteaux de filtration sont représentatifs de 100% de la matière du fond (radeaux ayant plongé/coulé au fond du bécher).

### 6.4.c. Résultats de l'essai 2

### 6.4.c.1. Concernant le comprimé de l'exemple 2

Les résultats de l'essai 2 obtenus pour le comprimé de l'exemple 2 sont présentés dans le tableau 9 infra.

**Tableau 9**

| **1. COMPRIME DE L'EXEMPLE 2** | | | |
|---|---|---|---|
| *tare essai [g]* | *poids bécher+ pe [g]* | *prise d'essai [g]* | *radeau [g]*/*comprimé 1700 mg* |
| 62.7064 | 64.3702 | **1.6638** | **10.64** |
| *tare filtre [g]* | *poids filtre [g]* + *radeau [g]* | *radeau [g]* | |
| 29.3763 | 39.7875 | **10.4112** | |

| **2. COMPRIME DE L'EXEMPLE 2** | | | |
|---|---|---|---|
| *tare essai [g]* | *poids bécher+ pe [g]* | *prise d'essai [g]* | *radeau [g]*/*comprimé 1700 mg* |
| 65.936 | 67.5998 | **1.6638** | **9.87** |
| *tare filtre [g]* | *poids filtre [g]* + *radeau Í0l* | *radeau [g]* | |
| 31.0268 | 40.6909 | **9.6641** | |

| **3. COMPRIME DE L'EXEMPLE 2** | | | |
|---|---|---|---|
| *tare essai [g]* | *poids bécher+ pe [g]* | *prise d'essai [g]* | *radeau [g]*/*comprimé 1700 mg* |
| 64.4686 | 66.1786 | **1.71** | **10.41** |
| *tare filtre [g]* | *poids filtre [g]* + *radeau [g]* | *radeau [g]* | |
| 29.3521 | 39.8235 | **10.4714** | |

| **4. COMPRIME DE L'EXEMPLE 2** | | | |
|---|---|---|---|
| *tare essai [g]* | *poids bécher+ pe [g]* | *prise d'essai [g]* | *radeau [g]*/*comprimé 1700 mg* |
| 65.2338 | 66.9504 | **1.7166** | 11.25 |
| *tare filtre [g]* | *poids filtre [g]* + *radeau [g]* | *radeau [g]* | |
| 29.2572 | 40.6163 | **11.3591** | |

| **5. COMPRIME DE L'EXEMPLE 2** | | | |
|---|---|---|---|
| *tare essai [g]* | *poids bécher+ pe [g]* | *prise d'essai [g]* | *radeau [g]*/*comprimé 1700 mg* |
| 66.6844 | 68.3715 | **1.6871** | **11.08** |
| *tare filtre [g]* | *poids filtre [g]* + *radeau [g]* | *radeau [g]* | |
| 31.2557 | 42.2489 | **10.9932** | |

| **6. COMPRIME DE L'EXEMPLE 2** | | | |
|---|---|---|---|
| *tare essai [g]* | *poids bécher+ pe [g]* | *prise d'essai [g]* | *radeau [g]*/*comprimé 1700* mg |
| 64.5639 | 66.2441 | **1.6802** | **11.3** |
| *tare filtre [g]* | *poids filtre [g]* + *radeau [g]* | *radeau [g]* | |
| 29.0679 | 40.2412 | **11.1733** | |

| **7. COMPRIME DE L'EXEMPLE 2** | | | |
|---|---|---|---|
| *tare essai [g]* | *poids bécher+ pe [g]* | *prise d'essai [g]* | *radeau [g]*/*comprimé 1700mg* |
| 64.4018 | 66.0981 | **16936** | **11.28** |
| *tare filtre [g]* | *poids filtre [g]* + *radeau [g]* | *radeau [g]* | |
| 31.0518 | 42.3056 | **11.2538** | |

| **8. COMPRIME DE L'EXEMPLE 2** | | | |
|---|---|---|---|
| *tare essai [g]* | *poids bécher+ pe [g]* | *prise d'essai [g]* | *radeau [g]*/*comprimé 1700 mg* |
| 67.5091 | 69.2093 | **1.7002** | **11.28** |
| *tare filtre [g]* | *poids filtre [g]* + *radeau [g]* | *radeau [g]* | |
| 28.9516 | 40.234 | **11.2824** | |

| **9. COMPRIME DE L'EXEMPLE 2** | | | |
|---|---|---|---|
| *tare essai [g]* | *poids bécher+ pe [g]* | *prise d'essai [g]* | *radeau [g]*/*comprimé 1700mg* |
| 66.2946 | 68.0075 | **1.7129** | **11.68** |
| *tare filtre [g]* | *poids filtre [g]* + *radeau [g]* | *radeau [g]* | |
| 31.0479 | 42.8129 | **11.765** | |

| **10. COMPRIME DE L'EXEMPLE 2** | | | |
|---|---|---|---|
| *tare essai [g]* | *poids bécher+ pe [g]* | *prise d'essai [g]* | *radeau [g]*/*comprimé 1700 mg* |
| 64.5185 | 66.2208 | **1.7023** | **11.13** |
| *tare filtre [g]* | *poids filtre [g]* + *radeau [g]* | *radeau [g]* | |
| 30.9422 | 42.0824 | **11.1402** | |
| | | | |
| | | | ***radeau [g]*/*comprimé 1700 mg*** |
| | | **n** | 10 |
| | | **moyenne** | **10.99** |
| | | **écart-type** | 0.53 |
| | | **rsd** % | 4.83 |

Tel qu'indiqué dans le tableau 9 supra, le poids moyen du radeau de 11,0 g est très répétable/reproductible (coefficient de variation de 4,8% sur 10 essais avec un min de 9,9 g et un max de 11,3 g).

### 6.4.c.2. Concernant le produit de référence

Les résultats de l'essai 2 obtenus pour le produit de référence sont présentés dans le tableau 10 infra.

**Tableau 10**

| 1. **PRODUIT DE REFERENCE** | | | |
|---|---|---|---|
| *tare essai [g]* | *poids bécher+ pe [g]* | *prise d'essai [g]* | *fond [g]*/*comprimé 1700 mg* |
| 62.7069 | 64.2749 | **1.568** | **10.65** |
| *tare filtre [g]* | *poids filtre [g]* + *fond [g]* | *fond [g]* | |
| 31.0473 | 40.8715 | **9.8242** | |

| **2. PRODUIT DE REFERENCE** | | | |
|---|---|---|---|
| *tare essai [g]* | *poids bécher+ pe [g]* | *prise d'essai [g]* | *fond [g]*/*comprimé 1700 mg* |
| 65.9368 | 67.4925 | **1.5557** | **12.37** |
| *tare filtre [g]* | *poids filtre [g]* + *fond [g]* | *fond [g]* | |
| 29.0633 | 40.3870 | **11.3237** | |

| **3. PRODUIT DE REFERENCE** | | | |
|---|---|---|---|
| *tare essai [g]* | *poids bécher+ pe [g]* | *prise d'essai [g]* | *fond [g]*/*comprimé 1700 mg* |
| 64.4696 | 66.0513 | **1.5817** | **8.44** |
| *tare filtre [g]* | *poids filtre [g]* + *fond [g]* | *fond [g]* | |
| 29.3518 | 37.2000 | **7.8482** | |

| **4. PRODUIT DE REFERENCE** | | | |
|---|---|---|---|
| *tare essai [g]* | *poids bécher+ pe [g]* | *prise d'essai [g]* | *fond [g]*/*comprimé 1700 mg* |
| 65.2343 | 66.7961 | **1.5618** | **10.16** |
| *tare filtre [g]* | *poids filtre [g]* + *fond [g]* | *fond [g]* | |
| 29.3713 | 38.7029 | **9.3316** | |

| **5. PRODUIT DE REFERENCE** | | | |
|---|---|---|---|
| *tare essai [g]* | *poids bécher+ pe [g]* | *prise d'essai [g]* | *fond [g]*/*comprimé 1700 mg* |
| 66.6846 | 68.2714 | **1.5868** | **13.46** |
| *tare filtre [g]* | *poids filtre [g]* + *fond [g]* | *fond [g]* | |
| 31.2557 | 43.8181 | **12.5624** | |

| **6. PRODUIT DE REFERENCE** | | | |
|---|---|---|---|
| *tare essai [g]* | *poids bécher+ pe [g]* | *prise d'essai [g]* | *fond [g]*/*comprimé 1700 mg* |
| 64.5642 | 66.1568 | **1.5926** | **11.85** |
| *tare filtre [g]* | *poids filtre [g]* + *fond [g]* | *fond [g]* | |
| 28.95 | 40.0525 | **11.1025** | |

| **7. PRODUIT DE REFERENCE** | | | |
|---|---|---|---|
| *tare essai [g]* | *poids bécher+ pe [g]* | *prise d'essai [g]* | *fond [g]*/*comprimé 1700 mg* |
| 64.4021 | 66.039 | **1.6369** | **13.16** |
| *tare filtre [g]* | *poids filtre [g]* + *fond [g]* | *fond [g]* | |
| 31.0507 | 43.7227 | **12.672** | |

| **8. PRODUIT DE REFERENCE** | | | |
|---|---|---|---|
| *tare essai [g]* | *poids bécher+ pe [g]* | *prise d'essai [g]* | *fond [g]*/*comprimé 1700 mg* |
| 67.5101 | 69.0793 | **1.5692** | **12.35** |
| *tare filtre [g]* | *poids filtre [g]* + *fond [g]* | *fond [g]* | |
| 29.2557 | 40.6519 | **11.3962** | |

| **9. PRODUIT DE REFERENCE** | | | |
|---|---|---|---|
| *tare essai [g]* | *poids bécher+ pe [g]* | *prise d'essai [g]* | *fond [g]*/*comprimé 1700 mg* |
| 66.2956 | 67.8667 | **1.5711** | **14.17** |
| *tare filtre [g]* | *poids filtre [g]* + *fond [g]* | *fond [g]* | |
| 31.0292 | 44.1217 | **13.0925** | |

| **10. PRODUIT DE REFERENCE** | | | |
|---|---|---|---|
| *tare essai [g]* | *poids bécher+ pe [g]* | *prise d'essai [g]* | *fond [g]*/*comprimé 1700 mg* |
| 64.5199 | 66.1093 | **1.5894** | **17.70** |
| *tare filtre [g]* | *poids filtre [g]* + *fond [g]* | *fond [g]* | |
| 30.9404 | 47.4864 | **16.546** | |
| | | | |
| | | | ***fond [g]*/*comprimé 1700 mg*** |
| | | **n** | 10 |
| | | **moyenne** | **12.43** |
| | | **écart-type** | 2.52 |
| | | **rsd %** | 20.24 |

Comme indiqué dans le tableau 10 supra, le poids moyen du fond (radeau plongé/coulé) est de 12,43 g pour un comprimé de référence de 1700 mg. Le poids moyen du « radeau » n'est pas répétable/reproductible (coefficient de variation de 20,2% sur 10 essais avec un min de 8,4 g et un max de 17,7 g).

### 6.4.d. Conclusion de l'essai 2

Les principaux enseignements de l'essai 2 sont récapitulés dans le tableau 11 infra.

**Tableau 11**

| | **Comprimé de l'exemple 2** | **Produit de référence (GAVISCON^{®})** |
|---|---|---|
| **Poids moyen (n=10)** | 10,99g | 12,43g |
| **Ecart-type** | 0,53 | 2,52 |
| **%RSD** | 4,83 | 20,24 |

Même si les poids des radeaux obtenus apparaissent *prima facie* similaires, il est important de noter que l'ajout de poudre d'écorce interne d'orme rouge permet une meilleure répétabilité/reproductibilité, à savoir plus de cinq fois supérieure.

### 6.5. Essais rhéologiques

### 6.5.a. Mode opératoire - formation du gel

Peser la poudre de 3 comprimés broyés dans un bécher 150 ml forme haute. Sous agitation magnétique vitesse 3, ajouter 50,0 ml d'acide chlorhydrique 0,1 N/22°C et augmenter la vitesse d'agitation à 5 dès le début du versement des premiers millilitres d'acide chlorhydrique. Agiter pendant 30 secondes (fin dégagement CO₂). Ajouter 2,5 ml acide chlorhydrique 1.0N/22°C en 15 secondes et continuer l'agitation pendant 15 secondes à la vitesse 5. Baisser le réglage sur la vitesse 3 puis continuer l'agitation encore exactement pendant 30 secondes. Retirer le barreau aimanté du bécher puis poursuivre l'agitation à 400 rpm en utilisant l'agitateur secoueur orbital IKA pendant exactement 5 minutes. Laisser reposer pendant 2 heures à 22°C puis effectuer les essais rhéologiques suivants les uns après les autres, puis mesurer le pH à la fin des essais.

A noter que, pour éviter le phénomène d'évaporation, nous avons couvert les bécher d'essai pendant toute la phase de repos avec un para film.

### 6.5.b. Essai 3 (durée d'écoulement)

### 6.5.b.1 Mode opératoire

Afin de mesurer la durée d'écoulement, une coupe d'écoulement/de viscosité ERICHSEN ISO 2431 Nr.6 04/331 a été utilisée.

En utilisant un gant de protection, obturer l'orifice d'écoulement avec l'index puis verser le gel en oscillant et tournant légèrement le bécher manuellement. Libérer l'orifice d'écoulement en déclenchant au même moment le chronomètre. La durée d'écoulement en secondes est notée au moment où l'ouverture de l'orifice reste définitivement visible, donc ne s'obture plus par présence de gel. Nous exprimons les résultats comme volume d'écoulement par minute (ml/min). A noter que cette technique est utilisée dans un but purement comparatif car une technique de cette nature est normalement employée pour les liquides newtoniens.

### 6.5.b.2. Résultats de l'essai 3

### 6.5.b.2.a Concernant le comprimé de l'exemple 2

Les résultats obtenus pour le comprimé de l'exemple 2 sont présentés dans le tableau 12 infra.

**Tableau 12**

| **Comprimé de l'exemple 2** | | | |
|---|---|---|---|
| **Essai n°** | **durée d'écoulement en sec.** | **volume du gel (ml)** | **volume d'écoulement (ml/min)** |
| 1 | 900 | 50 | 3 |
| 2 | 585 | 50 | 5 |
| 3 | 565 | 50 | 5 |
| 4 | 690 | 50 | 4 |
| 5 | 570 | 50 | 5 |
| 6 | 827 | 50 | 4 |
| 7 | 607 | 50 | 5 |
| 8 | 868 | 50 | 3 |
| 9 | 420 | 50 | 7 |
| 10 | 565 | 50 | 5 |
| **moyenne** | | | **4.8** |
| **écart-type** | | | **1.1** |
| **rsd** % | | | **24** |

L'écoulement ne se fait que par intervalles sous forme d'un goutte à goutte plus au moins régulier et la vitesse d'écoulement n'est pas linéaire (de plus en plus longue du début jusqu'à la fin).

### 6.5.b.2.b. Concernant le produit de référence

Les résultats obtenus pour le produit de référence sont présentés dans le tableau 13 infra.

**Tableau 13**

| **Produit de référence** | | | |
|---|---|---|---|
| **Essai n°** | **durée d'écoulement en sec.** | **volume du gel (ml)** | **volume d'écoulement (ml/min)** |
| 1 | 4 | 50 | 750 |
| 2 | 5 | 50 | 600 |
| 3 | 5 | 50 | 600 |
| 4 | 5 | 50 | 600 |
| 5 | 5 | 50 | 600 |
| 6 | 5 | 50 | 600 |
| 7 | 4 | 50 | 750 |
| 8 | 5 | 50 | 600 |
| 9 | 5 | 50 | 600 |
| 10 | 2 | 50 | 600 |
| **moyenne** | | | **630** |
| **écart-type** | | | **63.2** |
| **rsd** % | | | **10.0** |

Dans les conditions de l'essai, le gel se comporte comme un liquide, l'écoulement est très rapide, à une vitesse régulière.

### 6.5.b.3. Conclusion de l'essai 3

Cet essai comparatif permet de démontrer la bonne tenue du radeau gélifié formé à partir du comprimé de l'exemple 2 par rapport au radeau gélifié formé à partir du produit de référence, lequel se liquéfie facilement pendant les manipulations.

### 6.5.c. Essai 4 (mesure de la consistance du radeau)

### 6.5.c.1. Mode opératoire

Pour réaliser cet essai, un consistomètre de Bostwick (Endecotts Ltd. Royaume Uni Réf. : ZXCON-CON1 N° d'inventaire : 6089) est utilisé. Ce consistomètre permet de déterminer aisément la consistance de produits comme les confitures, les miels, les gels et les produits à viscosité élevé. Le principe de fonctionnement du consistomètre est de remplir un espace avec le produit à tester. Le compartiment de gauche contient 50 ml de gel obtenu à partir du comprimé de l'exemple 2 (ou à partir du produit de référence) qui est retenu par une barrière montée sur ressort. A la suite d'un nivellement précis, ce montage permet l'ouverture instantané de la barrière pour libérer le gel (radeau) qui en s'étalant évolue à 22°C sur le parcours en pente du compartiment de droite, long de 23,5 cm avec une résolution de 0,5 cm. Quand la texture du produit le permet, nous avons pris des mesures après 15 et 30 secondes.

### 6.5.c.2 Résultats de l'essai 4

### 6.5.c.2.a. Concernant le comprimé de l'exemple 2

Les résultats obtenus pour le comprimé de l'exemple 2 sont présentés dans le tableau 14 infra.

**Tableau 14**

| **Comprimé de l'exemple 2** | | 100% | | 100% | | 0% | |
|---|---|---|---|---|---|---|---|
| Gel n° | distance d'écoulement [cm/15 sec.] | cm | sec. | cm | sec. | cm max. | sec. |
| 1 | 3.25 | 3.25 | 15 | 3.5 | 30 | | |
| 2 | 4.50 | 4.50 | 15 | 4.75 | 30 | | |
| 3 | 3.50 | 3.50 | 15 | 4.00 | 30 | | |
| 4 | 4.00 | 4.00 | 15 | 4.50 | 30 | | |
| 5 | 4.50 | 4.50 | 15 | 4.75 | 30 | | |
| 6 | 3.50 | 3.50 | 15 | 4.00 | 30 | | |
| 7 | 3.75 | 3.75 | 15 | 4.00 | 30 | | |
| 8 | 3.50 | 3.50 | 15 | 4.00 | 30 | | |
| 9 | 4.50 | 4.50 | 15 | 5.00 | 30 | | |
| 10 | 4.25 | 4.25 | 15 | 4.50 | 30 | | |
| moyenne | 3.9 | | | 4.3 | | | |
| écart-type | 0.5 | | | 0.5 | | | |
| rsd % | 12.4 | | | 10.9 | | | |

Le gel obtenu à partir du comprimé de l'exemple 2 est très consistant et nous avons mesuré la distance d'écoulement pour 100% des gels obtenus à partir du comprimé de l'exemple 2 à 15 sec. et à 30 secondes. La répétabilité/reproductibilité des séries de mesures à 15 et 30 secondes est satisfaisante.

### 6.5.c.2.b. Concernant le produit de référence

Les résultats obtenus pour le produit de référence sont présentés dans le tableau 15 infra.

**Tableau 15**

| **Produit de référence** | | | 70% | | 50% | | 50% | |
|---|---|---|---|---|---|---|---|---|
| Gel n° | distance d'écoulement [cm/15 sec.] | | cm | sec. | cm | sec. | cm max. | sec. |
| 1 | 20.0 | 20.0 | 20 | 15 | 23 | 30 | | |
| 2 | 18.0 | 18.0 | 18 | 15 | 21.5 | 30 | | |
| 3 | 16.5 | 16.5 | 16.5 | 15 | 18 | 30 | | |
| 4 | 16.5 | 16.5 | 16.5 | 15 | 19 | 30 | | |
| 5 | 21.0 | 21.0 | 21 | 15 | | | 23.5 | 21 |
| 6 | | 29.4 * | | | | | 23.5 | 12 |
| 7 | 21.0 | 21.0 | 21 | 15 | | | 23.5 | 22 |
| 8 | 20.5 | 20.5 | 20.5 | 15 | 23 | 30 | | |
| 9 | | 32.0 * | | | | | 23.5 | 11 |
| 10 | | 50.4 * | | | | | 23.5 | 7 |
| n | 7 | 10 | | | | | | |
| moyenne | 19.1 | 24.5 * | | | | | | |
| écart-type | 2.0 | 10.4 * | | | | | | |
| rsd % | 10.6 | 42.5 * | | | | | | |

Dans les conditions d'essai, le gel obtenu à partir du produit de référence est très fluide. Malgré sa fluidité, nous avons pu mesurer la distance d'écoulement inférieure à la distance maximale réelle de 23,5 cm pour 70% des gels obtenus à partir du produit de référence comprimé à 15 secondes et pour 50 % des gels à 30 secondes. Pour pouvoir comparer la dispersion statistique des mesures du produit aux autres, nous avons créé des distances maximales interprétées pour trois mesures hors plage de parcours (nombres suivis d'une astérisque) en se basant sur le temps (inférieur à 15 secondes) qui leur a été nécessaire pour parcourir les 23,5 cm.

Le produit de référence génère donc un gel peu consistant et de consistance peu répétable (coefficient de variation de 42,5% sur 10 essais).

### 6.5.c.3. Conclusion

Dans les conditions de l'essai 4, le comprimé de l'exemple 2 donne un gel de consistance largement supérieure à la tenue du gel produite par le produit de référence. Le gel formé par ce dernier ne peut être sollicité mécaniquement sans qu'il se déstructure rapidement en se liquéfiant irréversiblement, ce qui représente un problème mécanique majeur.

### 6.5.d. Essai 5 (viscosité du gel)

### 6.5.d.1. Mode opératoire

Toutes les mesures de cet essai ont été réalisés avec un viscosimètre de Brookfield sur support Helipath et mobile T-bar. La viscosité du gel a été mesurée à 22°C en mode HELIPATH- statique en utilisant le mobile S96 à une vitesse de rotation de 50 rpm après un temps de stabilisation de 60 secondes. Dans les résultats, nous exprimons la viscosité en centipoise cP (1cP=1 mPa.s).

### 6.5.d.2. Résultats de l'essai 5

Les résultats de l'essai 5, pour le comprimé de l'exemple 2 et le produit de référence sont présentés dans le tableau 16 infra.

**Tableau 16**

| **Comprimé de l'exemple 2** | | | **Produit de référence** | | |
|---|---|---|---|---|---|
| Gel n° | cP | torsion % | Gel n° | cP | torsion % |
| 1 | 4500 | 24.0 | 1 | 206 | 1.2 |
| 2 | 4818 | 26.0 | 2 | 243 | 1.3 |
| 3 | 4260 | 23.0 | 3 | 263 | 1.3 |
| 4 | 4500 | 25.0 | 4 | 300 | 1.6 |
| 5 | 4218 | 24.0 | 5 | 225 | 1.2 |
| 6 | 4550 | 24.0 | 6 | 168 | 0.9 |
| 7 | 4600 | 25.0 | 7 | 244 | 1.3 |
| 8 | 4700 | 25.0 | 8 | 281 | 1.5 |
| 9 | 4400 | 24.0 | 9 | 206 | 1.0 |
| 10 | 4450 | 24.0 | 10 | 187 | 1.0 |
| moyenne | 4500 | 24.4 | moyenne | 232 | 1.2 |
| écart-type | 184 | 0.8 | écart-type | 42 | 0.2 |
| rsd % | 4 | 3.5 | rsd % | 18 | 18.0 |

A noter que la mesure de la viscosité a été faite dans le but de comparer des produits dans les mêmes conditions opératoires (type de mobile, vitesse de rotation par minutes, etc.).

### 6.5.d.3. Conclusion

Le gel obtenu à partir du comprimé de l'exemple 2 (4500 cP) montre une viscosité plus de dix-neuf fois supérieure à celle du gel obtenu à partir du produit de référence (232 cP). La répétabilité/reproductibilité des valeurs obtenues à partir du comprimé de l'exemple 2 est bien meilleure que celle obtenue à partir du produit de référence (Coefficient de variation de 4% pour le comprimé de l'exemple 2 contre 18% pour le produit de référence).

### 6.6. Essai 6 (détermination du pH du gel à 22°C)

### 6.6.a. Mode opératoire

La mesure du pH à 22°C a été faite en plongeant l'électrode double dans le gel puis en remuant pendant quelques secondes l'électrode, nous attendons la stabilisation du signal en mode statique (sans agitation).

### 6.6.b. Résultat de l'essai 6

Les résultats de l'essai 6, pour le comprimé de l'exemple 2 et le produit de référence sont présentés dans le tableau 17 infra, lequel montre les valeurs du pH après stabilisation du signal.

**Tableau 17**

| **Comprimé de l'exemple 2** | | **Produit de référence** | |
|---|---|---|---|
| Gel n° | pH 22°C | Gel n° | pH 22°C |
| 1 | 6.86 | 1 | 7.12 |
| 2 | 6.88 | 2 | 7.08 |
| 3 | 6.77 | 3 | 7.13 |
| 4 | 6.72 | 4 | 7.15 |
| 5 | 6.73 | 5 | 7.05 |
| 6 | 6.78 | 6 | 7.02 |
| 7 | 6.69 | 7 | 7.01 |
| 8 | 6.69 | 8 | 6.95 |
| 9 | 6.61 | 9 | 7.05 |
| 10 | 6.7 | 10 | 6.98 |
| moyenne | 6.7 | moyen ne | 7.1 |
| écart-type | 0.1 | écart-type | 0.1 |
| rsd % | 1.2 | rsd % | 0.9 |

### 6.6.c. Conclusion de l'essai 6

Dans les conditions de l'essai 6, les valeurs des deux séries sont reproductibles. Le gel obtenu à partir du comprimé de l'exemple 2 montre dans ces conditions un pH moyen proche de la neutralité (bien que légèrement acide (pH 6,7)) par rapport au gel obtenu à partir du produit de référence qui a un pH moyen de 7,1.

### 6.7. Conclusion de l'exemple 6

L'exemple 6 a permis de mettre en avant la supériorité de la composition selon l'invention par rapport au produit de référence (GAVISCON^{®} menthe, comprimé à croquer 1700 mg), notamment en ce qui concerne les propriétés suivantes :
- Doublement du volume du radeau (gel) avec reproductibilité de formation quatre fois supérieure, et stabilité dans le temps nettement améliorée,
- Poids du radeau (gel) obtenu similaire mais avec une reproductibilité plus de cinq fois supérieure pour la composition selon l'invention, et
- Propriétés rhéologiques du radeau améliorées en matière d'écoulement, de consistance [avec une répétabilité/reproductibilité nettement supérieure pour la composition selon l'invention], et en termes de viscosité [également avec une répétabilité/reproductibilité bien supérieure pour la composition selon l'invention], démontrant la bonne tenue du radeau gélifié obtenu à partir de la composition selon l'invention.

Par conséquent, il convient d'admettre que la composition selon l'invention permet la formation d'un radeau gélifié possédant des propriétés physico-chimiques et rhéologiques nettement améliorées par rapport à celles du produit de référence. Ceci est attribuable, selon toute vraisemblance, à la présence de poudre d'écorce interne d'orme rouge dans la composition selon l'invention.

Ces résultats *in vitro* sont tout à fait extrapolables aux radeaux gélifiés formés en milieu gastrique *(in vivo).*

### Exemple 7 : Analyse comparative (en microscopie électronique) de la microstructure de deux radeaux gélifiés (gels) : celui obtenu à partir du comprimé de l'exemple 2 et celui obtenu à partir du produit de référence testé à l'exemple 6 (GAVISCON^{®})

### 7.1 Matériels et Méthodes

1) Préparation des gels : mode opératoire identique à celui décrit dans l'exemple 6 supra.
2) Analyses de la microstructure des gels par Cryo-mEB : microscopie électronique à balayage en mode d'observation « cryo » des échantillons.

Les échantillons ont été déposés sur des supports, vitrifiés dans l'azote pâteux (azote liquide sous vide), maintenus à -140 °C et sous vide pendant le transfert dans la chambre de préparation de la platine cryogénique Quorum PP3010T. Dans cette chambre, maintenue sous vide secondaire, les échantillons ont été ensuite fracturés en partie pour accéder à structure interne du gel. L'eau libre a été sublimée à -90°C pendant 10 min sous vide. Puis à -140°C, les échantillons ont été recouverts de platine (5mA pendant 15s) sous argon. Les échantillons sont ensuite transférés dans la chambre du Microscope Electronique à Balayage (Hitachi SU8230) et observés à -140°C.

### 7.2 Résultats

L alginate ou le composé formant le gel est visible en blanc sur fond gris/noir qui correspond aux espaces occupés par l'eau sublimée.

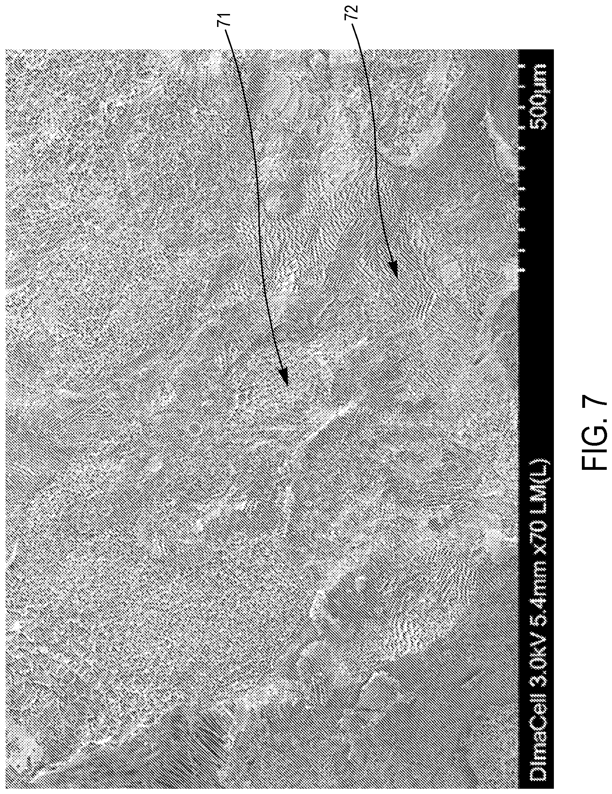

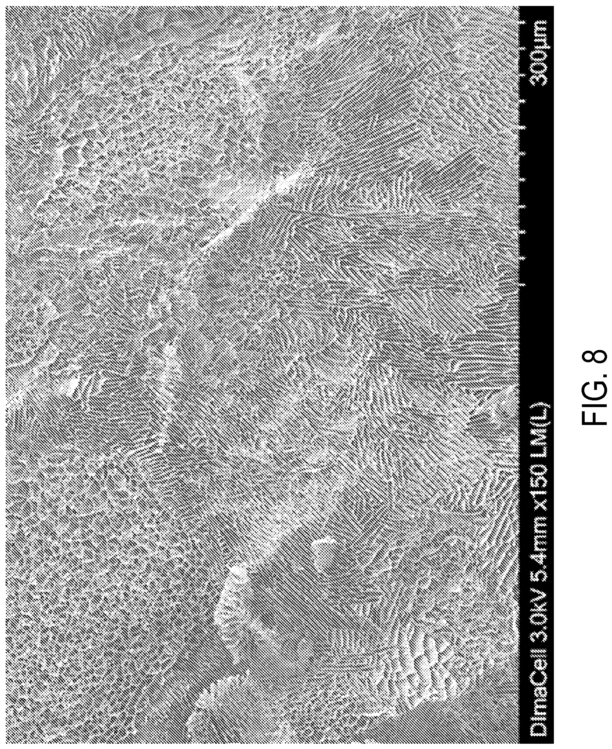

Les maillages des composés du gel (microstructure) peuvent alors être comparés. L'on peut observer : des alvéoles en « boîte à oeufs » (alginates +++, eau + ; cf. référence numérique 71 en figure 7) et des alvéoles en « bande » (alginate +, eau +++ ; cf. référence numérique 72 en figure 8)

### 7.2.a Gel obtenu à partir du produit de référence

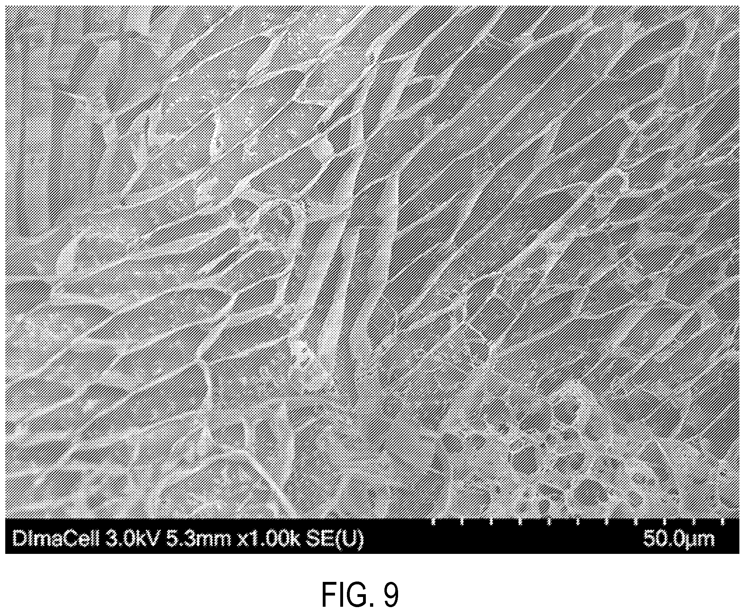

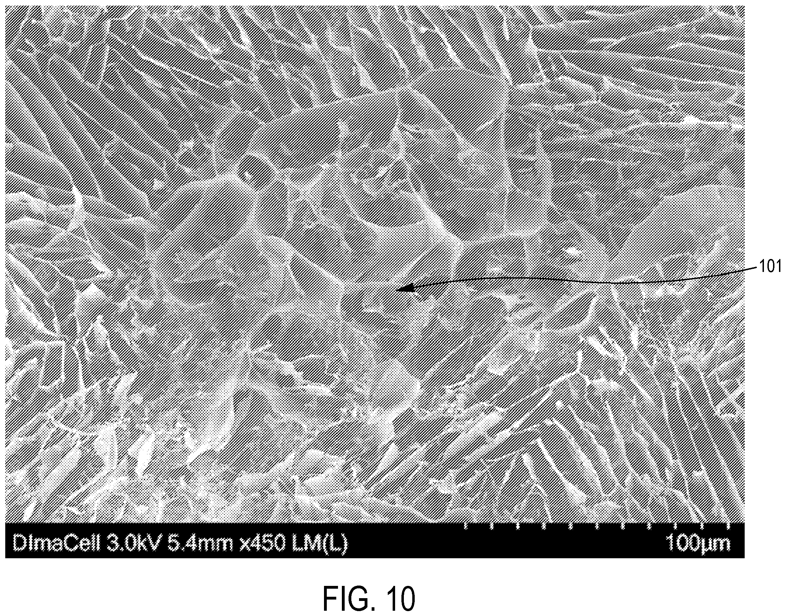

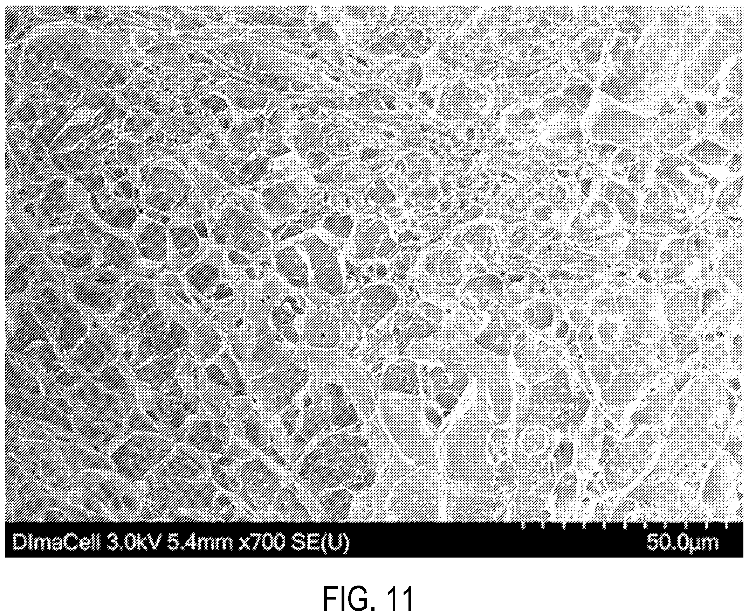

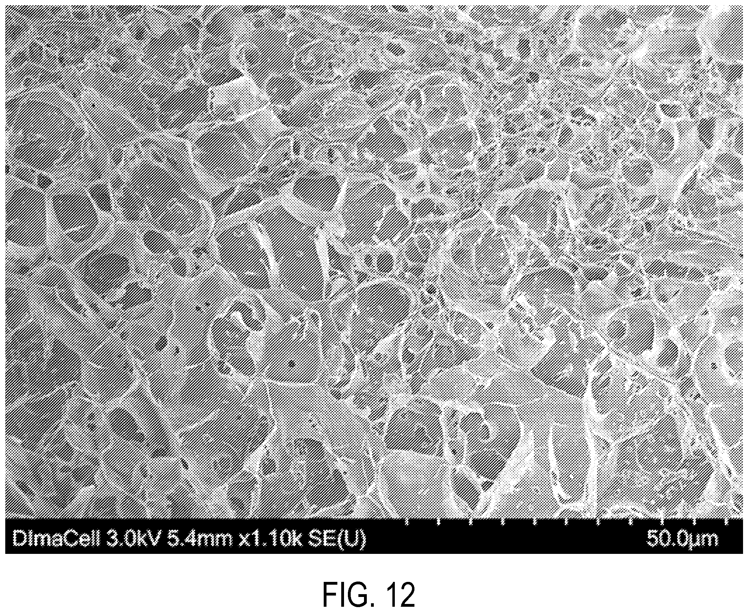

Sur les figures 7, 8 et 9, l'on note la présence importante des « bandes » susvisées, traduisant le fait qu'une part importante du gel est très aqueuse. Les îlots de « phase gel » sont dispersés dans cette trame aqueuse et montrent une microstructure arrangée en « boîte à oeufs » (cf. figure 10, référence numérique 101), mais un peu chaotique, les parois gélifiées semblant fragiles (cf. figures 11 et 12).

### 7.2.b Gel obtenu à partir du comprimé de l'exemple 2

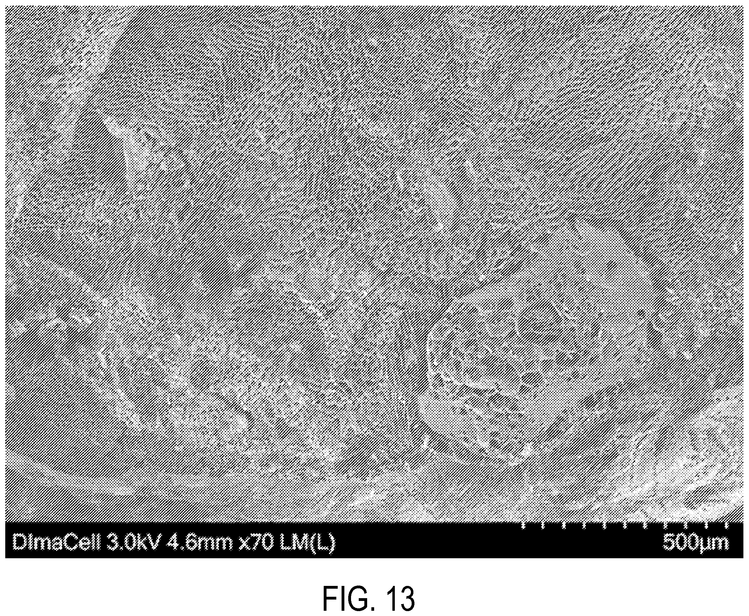

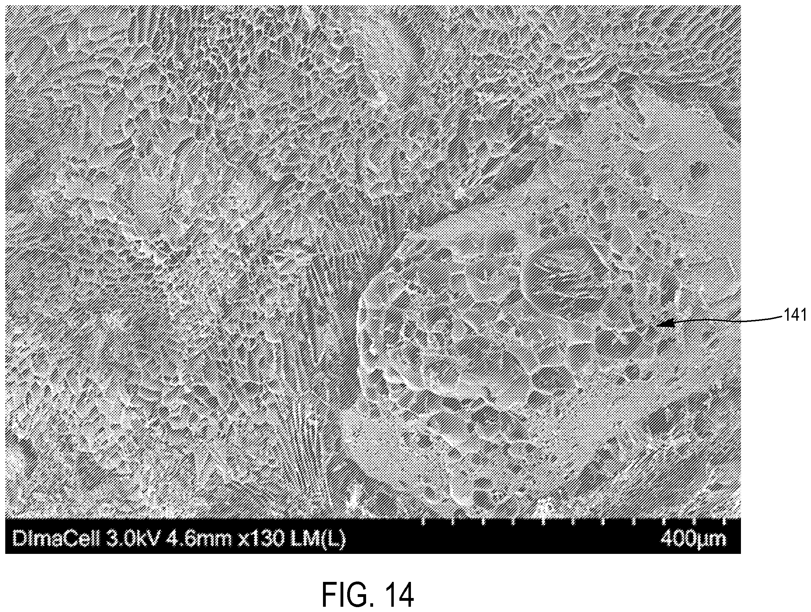

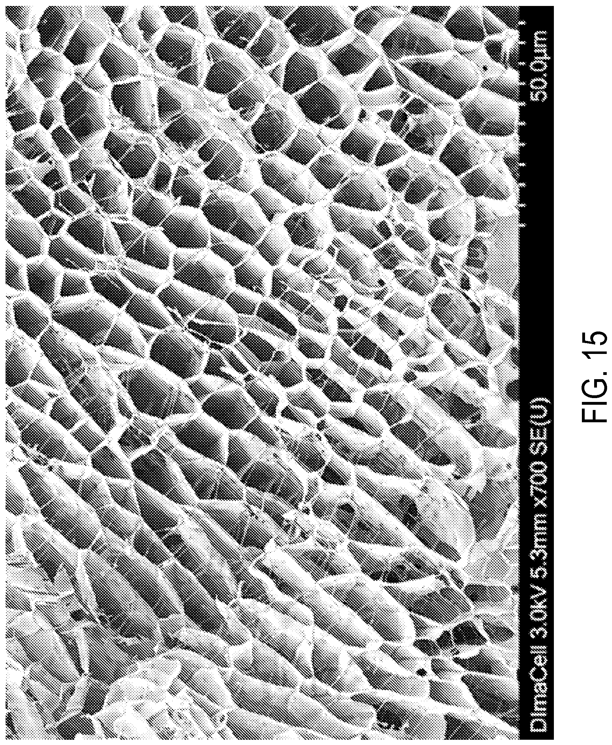

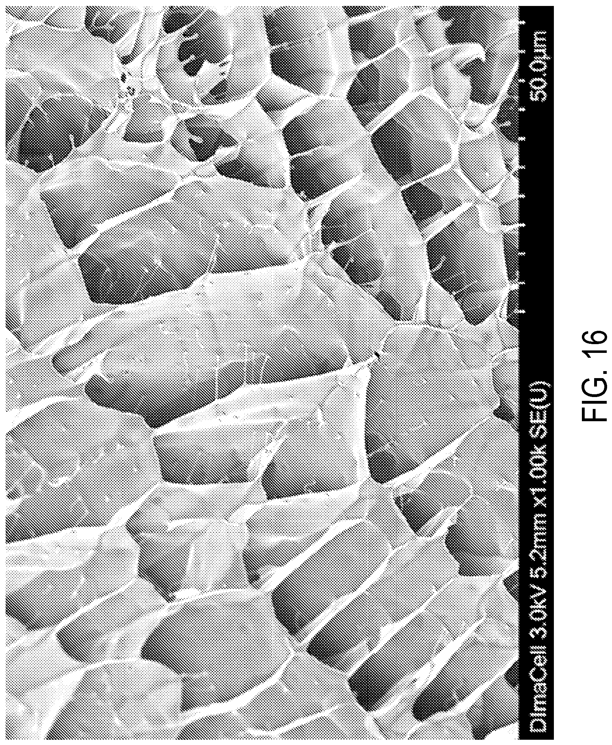

Comme cela est visible sur les figures 13 et 14, les îlots de « phase gel » (cf. figure 14, référence numérique 141) sont dispersés dans une trame plus gélifiée (moins riche en eau que le produit de référence, plus structurée). Les alvéoles du gel sont régulières et semblent plus résistantes (cf. figure 15), plus élastiques également (cf. figure 16).

### 7.3. Conclusion

L'analyse en microscopie électronique révèle que l'ajout de poudre d'écorce d'orme rouge améliore nettement la microstructure du gel/radeau gélifié obtenu. Ceci permet, selon toute vraisemblance, d'expliquer - au moins en partie - la supériorité du radeau gélifié obtenu à partir de la composition selon l'invention en termes de propriétés physico-chimiques et rhéologiques (cf. exemple 6 supra) par rapport aux radeaux gélifiés produits par les préparations pharmaceutiques de type GAVISCON^{®}.

## Revendications

1. Composition administrable par voie orale à un individu humain ou animal, adaptée pour former, après ingestion, un radeau gélifié flottant à la surface du contenu gastrique dudit individu, ladite composition comprenant les ingrédients actifs suivants en quantités efficaces
a) une préparation à base d'écorce d'orme rouge *(Ulmus rubra Muhl.),* de préférence à base d'écorce interne d'orme rouge,
b) de l'acide alginique ou de l'alginate,
c) au moins un composé produisant un dégagement de dioxyde de carbone en milieu acide, tel que :
c.1) au moins un sel de bicarbonate, de préférence au moins un bicarbonate de métal alcalin, avantageusement du bicarbonate de sodium, du bicarbonate de potassium ou un mélange de ceux-ci, et/ou
c.2) au moins un sel de carbonate, de préférence au moins un carbonate de cation métallique divalent ou trivalent, préférablement au moins un carbonate de métal alcalino-terreux, avantageusement du carbonate de calcium, du carbonate de magnésium ou un mélange de ceux-ci, et
d) éventuellement au moins un agent de réticulation de l'acide alginique, de préférence au moins une source de cations métalliques divalents ou trivalents, préférablement au moins un sel de métal alcalino-terreux, avantageusement au moins un sel de calcium, un sel de magnésium ou un mélange des deux.

2. Composition selon la revendication précédente, dans laquelle c) comprend c.1) et/ou c.2).

3. Composition selon la revendication précédente comprenant l'ingrédient actif d) lorsque :
- c.2) est absent, ou
- c.2) est présent mais est différent d'au moins un carbonate de cation métallique divalent ou trivalent.

4. Composition selon la revendication 1, dans laquelle c) comprend c.1) et c.2).

5. Composition selon l'une des revendications précédentes, dans laquelle b) est un sel de métal alcalin ou alcalino-terreux d'alginate, de préférence un sel de métal alcalin d'alginate, préférablement un alginate de sodium et/ou un alginate de potassium, avantageusement un alginate de sodium.

6. Composition selon la revendication précédente, dans laquelle le rapport en masse a)/b) est compris entre 0,003 et 12.

7. Composition selon l'une des revendications précédentes, dans laquelle le rapport en masse a)/c.1) est compris entre 0,005 et 12.

8. Composition selon l'une des revendications précédentes, dans laquelle le rapport en masse a)/c.2) est compris entre 0,003 et 12.

9. Composition selon l'une des revendications précédentes, ladite composition comprenant les ingrédients actifs a), b), c.1) et c.2), et ladite composition comprenant, par unité de prise :
- en tant qu'ingrédient actif a) : de la poudre d'écorce d'orme rouge, de préférence d'écorce interne d'orme rouge, avantageusement dans une quantité comprise entre 5 et 600 mg,
- en tant qu'ingrédient actif b) : de l'alginate de sodium, de préférence dans une quantité comprise entre 50 et 1500 mg,
- en tant qu'ingrédient actif c.1) du bicarbonate de sodium, de préférence dans une quantité comprise entre 50 et 1000 mg,
- en tant qu'ingrédient actif c.2) du carbonate de calcium, de préférence dans une quantité comprise entre 50 et 500 mg.

10. Comprimé, de préférence comprimé à croquer, pastille, sachet, de préférence hermétique, stick, de préférence hermétique, solution, suspension buvable, gel ou sirop, comprenant la composition selon l'une des revendications 1 à 9.

11. Composition selon l'une des revendications 1 à 9 pour son utilisation en tant que médicament.

12. Composition selon l'une des revendications 1 à 9 pour son utilisation pour :
- le traitement ou la prévention des affections gastro-œsophagiennes et en particulier du reflux gastro-œsophagien (RGO) et/ou ses symptômes - tels que les remontées acides, les brûlures d'estomac, la digestion difficile, les indigestions, le pyrosis - de la gastrite, de la dyspepsie et des ulcères peptidiques,
- la délivrance prolongée ou ciblée d'ingrédient(s) actif(s) dans l'estomac et/ou à la jonction gastro-œsophagienne dudit individu, et/ou
- le soulagement, au niveau local, des muqueuses gastro-œsophagiennes irritées.

13. Utilisation d'une quantité efficace d'une préparation à base d'écorce d'orme rouge (*Ulmus rubra Muhl.),* de préférence à base d'écorce interne d'orme rouge, d'une quantité efficace 5 d'acide alginique ou d'alginate, d'une quantité efficace d'au moins un composé produisant un dégagement de dioxyde de carbone en milieu acide, et éventuellement au moins un agent de réticulation de l'acide alginique, pour préparer une composition adaptée pour former, après administration orale à un individu humain ou animal, un radeau gélifié flottant à la surface du contenu gastrique dudit individu. 10

14. Composition selon l'une des revendications 1 à 12, dans laquelle ladite préparation à base d'écorce d'orme rouge est une poudre d'écorce d'orme rouge, un extrait aqueux d'écorce d'orme rouge et/ou un extrait hydroglycériné d'écorce d'orme rouge, avantageusement une poudre d'écorce d'orme rouge et/ou un extrait aqueux d'écorce d'orme rouge, de manière 15 préférée une poudre d'écorce d'orme rouge ; de manière particulièrement préférée, l'écorce d'orme rouge étant l'écorce interne d'orme rouge.

15. Utilisation selon la revendication 13, dans laquelle ladite préparation à base d'écorce d'orme rouge est une poudre d'écorce d'orme rouge, un extrait aqueux d'écorce d'orme rouge 20 et/ou un extrait hydroglycériné d'écorce d'orme rouge, avantageusement une poudre d'écorce d'orme rouge et/ou un extrait aqueux d'écorce d'orme rouge, de manière préférée une poudre d'écorce d'orme rouge ; de manière particulièrement préférée, l'écorce d'orme rouge étant l'écorce interne d'orme rouge.

## Patentansprüche

1. Eine auf oralem Weg an ein menschliches oder tierisches Individuum verabreichbare Zusammensetzung, die angepasst ist, um nach der Einnahme ein geliertes Floß zu bilden, das auf der Oberfläche des Mageninhalts des Individuums schwimmt, wobei die Zusammensetzung die folgenden Wirkstoffe in wirksamen Mengen beinhaltet:
a) ein Präparat auf der Basis von Rotulmenrinde *(Ulmus rubra Muhl.),* vorzugsweise auf Basis von innerer Rotulmenrinde,
b) Alginsäure oder Alginat,
c) mindestens eine Verbindung, die in saurem Medium eine Freisetzung von Kohlendioxid produziert, wie z. B.:
c.1) mindestens ein Bicarbonatsalz, vorzugsweise mindestens ein Alkalimetallbicarbonat, vorteilhafterweise Natriumbicarbonat, Kaliumbicarbonat oder eine Mischung davon und/oder
c.1) mindestens ein Carbonatsalz, vorzugsweise mindestens ein Carbonat eines zweiwertigen oder dreiwertigen Metallkations, bevorzugt mindestens ein Erdalkalimetallcarbonat, vorteilhafterweise Calciumcarbonat, Magnesiumcarbonat oder eine Mischung davon und
d) gegebenenfalls mindestens ein Alginsäure-Vernetzungsmittel, vorzugsweise mindestens eine Quelle zweiwertiger oder dreiwertiger Metallkationen, bevorzugt mindestens ein Erdalkalimetallsalz, vorteilhafterweise mindestens ein Calciumsalz, ein Magnesiumsalz oder eine Mischung von beiden.

2. Zusammensetzung gemäß dem vorhergehenden Anspruch, wobei c) c.1) und/oder c.2) beinhaltet.

3. Zusammensetzung gemäß dem vorhergehenden Anspruch, beinhaltend den Wirkstoff d) wenn:
- c.2) fehlt oder
- c.2) vorhanden ist, sich jedoch von mindestens einem Carbonat eines zweiwertigen oder dreiwertigen Metallkations unterscheidet.

4. Zusammensetzung gemäß Anspruch 1, wobei c) c.1) und c.2) beinhaltet.

5. Zusammensetzung gemäß einem der vorhergehenden Ansprüche, wobei b) ein Alkali- oder Erdalkalimetallsalz von Alginat, vorzugsweise ein Alkalimetallsalz von Alginat, bevorzugt ein Natriumalginat und/oder ein Kaliumalginat, vorteilhafterweise ein Natriumalginat ist.

6. Zusammensetzung gemäß dem vorhergehenden Anspruch, wobei das Massenverhältnis a)/b) zwischen 0,003 und 12 liegt.

7. Zusammensetzung gemäß einem der vorhergehenden Ansprüche, wobei das Massenverhältnis a)/c.1) zwischen 0,005 und 12 liegt.

8. Zusammensetzung gemäß einem der vorhergehenden Ansprüche, wobei das Massenverhältnis a)/c.2) zwischen 0,003 und 12 liegt.

9. Zusammensetzung gemäß einem der vorhergehenden Ansprüche, wobei die Zusammensetzung die Wirkstoffe a), b), c.1) und c.2) beinhaltet und die Zusammensetzung pro Dosierungseinheit Folgendes beinhaltet:
- als Wirkstoff a): Pulver aus Rotulmenrinde, vorzugsweise innere Rotulmenrinde, vorteilhafterweise in einer Menge zwischen 5 und 600 mg,
- als Wirkstoff b): Natriumalginat, vorzugsweise in einer Menge zwischen 50 und 1 500 mg,
- als Wirkstoff c.1): Natriumbicarbonat, vorzugsweise in einer Menge zwischen 50 und 1 000 mg,
- als Wirkstoff c.2): Calciumcarbonat, vorzugsweise in einer Menge zwischen 50 und 500 mg.

10. Tablette, vorzugsweise eine Kautablette, eine Pastille, ein Beutel, vorzugsweise luftdicht, ein Stift, vorzugsweise luftdicht, eine Lösung, trinkbare Suspension, ein Gel oder Sirup, beinhaltend die Zusammensetzung gemäß einem der Ansprüche 1 bis 9.

11. Zusammensetzung gemäß einem der Ansprüche 1 bis 9 zur Verwendung als Medikament.

12. Zusammensetzung gemäß einem der Ansprüche 1 bis 9 zur Verwendung für Folgendes:
- die Behandlung oder die Prävention von gastroösophagealen Störungen und insbesondere gastroösophagealen Refluxkrankheiten (GERD) und/oder deren Symptomen - wie saurem Reflux, Sodbrennen, Verdauungsstörungen, Verdauungsbeschwerden, Pyrosis - Gastritis, Dyspepsie und peptischer Ulkus,
- die anhaltende oder gezielte Abgabe von Wirkstoff(en) an den Magen und/oder den gastroösophagealen Übergang des Individuums, und/oder
- die lokale Linderung der gereizten gastroösophagealen Schleimhäute.

13. Verwendung einer wirksamen Menge eines Präparats auf Basis von Rotulmenrinde *(Ulmus rubra Muhl.),* vorzugsweise auf Basis der inneren Rotulmenrinde, einer wirksamen Menge von Alginsäure oder Alginat, einer wirksamen Menge von mindestens einer Verbindung, die eine Freisetzung von Kohlendioxid in saurem Medium produziert und gegebenenfalls mindestens einem Alginsäure-Vernetzungsmittel, um eine Zusammensetzung herzustellen, die angepasst ist, um nach oraler Verabreichung an ein menschliches oder tierisches Individuum ein geliertes Floß zu bilden, das auf der Oberfläche des Mageninhalts des Individuums schwimmt.

14. Zusammensetzung gemäß einem der Ansprüche 1 bis 12, wobei das Präparat auf Basis von Rotulmenrinde ein Pulver aus Rotulmenrinde, ein wässriger Extrakt aus Rotulmenrinde und/oder ein Hydroglycerinextrakt aus Rotulmenrinde, vorteilhafterweise ein Pulver aus Rotulmenrinde und/oder ein wässriger Extrakt aus Rotulmenrinde, auf bevorzugte Weise ein Pulver aus Rotulmenrinde ist; wobei auf besonders bevorzugte Weise die Rotulmenrinde die innere Rotulmenrinde ist.

15. Verwendung gemäß Anspruch 13, wobei das Präparat auf Basis von Rotulmenrinde ein Pulver aus Rotulmenrinde, ein wässriger Extrakt aus Rotulmenrinde und/oder ein Hydroglycerinextrakt aus Rotulmenrinde, vorteilhafterweise ein Pulver aus Rotulmenrinde und/oder ein wässriger Extrakt aus Rotulmenrinde, auf bevorzugte Weise ein Pulver aus Rotulmenrinde ist; wobei auf besonders bevorzugte Weise die Rotulmenrinde die innere Rotulmenrinde ist.

## Claims

1. A composition that can be administered orally to a human or animal individual, suitable for forming, after ingestion, a gellified raft floating on the surface of the gastric contents of said individual, said composition comprising the following active ingredients in effective amounts:
a) a preparation based on slippery elm bark *(Ulmus rubra Muhl.),* preferably based on slippery elm inner bark,
b) alginic acid or alginate,
c) at least one compound producing a release of carbon dioxide in an acidic medium, such as:
c.1) at least one bicarbonate salt, preferably at least one alkali metal bicarbonate, advantageously sodium bicarbonate, potassium bicarbonate or a mixture thereof, and/or
c.2) at least one carbonate salt, preferably at least one divalent or trivalent metal cation carbonate, preferably at least one alkaline earth metal carbonate, advantageously calcium carbonate, magnesium carbonate or a mixture thereof, and
d) possibly at least one alginic acid cross-linking agent, preferably at least one source of divalent or trivalent metal cations, preferably at least one alkaline earth metal salt, advantageously at least one calcium salt, a magnesium salt or a mixture of the two.

2. The composition according to the preceding claim, wherein c) comprises c.1) and/or c.2).

3. The composition according to the preceding claim comprising the active ingredient d) when:
- c.2) is absent, or
- c.2) is present but is different by at least one divalent or trivalent metal cation carbonate.

4. The composition according to claim 1, wherein c) comprises c.1) and c.2).

5. The composition according to one of the preceding claims, wherein b) is an alkali or alkaline earth metal alginate salt, preferably an alkali metal alginate salt, preferably a sodium alginate and/or a potassium alginate, advantageously a sodium alginate.

6. The composition according to the preceding claim, wherein the a)/b) mass ratio is between 0.003 and 12.

7. The composition according to one of the preceding claims, wherein the a)/c.1) mass ratio is between 0.005 and 12.

8. The composition according to one of the preceding claims, wherein the a)/c.2) mass ratio is between 0.003 and 12.

9. The composition according to one of the preceding claims, said composition comprising the active ingredients a), b), c.1) and c.2), and said composition comprising, per intake unit:
- as active ingredient a): slippery elm bark powder, preferably slippery elm inner bark powder, advantageously in an amount of between 5 and 600 mg,
- as active ingredient b): sodium alginate, preferably in an amount of between 50 and 1500 mg,
- as active ingredient c.1) sodium bicarbonate, preferably in an amount of between 50 and 1000 mg,
- as active ingredient c.2) calcium carbonate, preferably in an amount of between 50 and 500 mg.

10. A tablet, preferably a chewable tablet, pill, sachet, preferably hermetically sealed, stick, preferably hermetically sealed, solution, drinkable suspension, gel or syrup, comprising the composition according to one of claims 1 to 9.

11. The composition according to one of claims 1 to 9 for its use as a medicine.

12. The composition according to one of claims 1 to 9 for its use in:
- the treatment or prevention of gastro-oesophageal affections and in particular gastro-oesophageal reflux (GOR) and/or the symptoms thereof - such as acid reflux, heartburn, digestion difficulties, indigestions, pyrosis - gastritis, dyspepsia and peptic ulcers,
- the prolonged or targeted delivery of active ingredient(s) into the stomach and/or into the gastro-oesophageal junction of said individual, and/or
- the relief, locally, of irritated gastro-oesophageal mucosa.

13. Use of an effective amount of a preparation based on slippery elm bark *(Ulmus rubra Muhl.),* preferably based on slippery elm inner bark, of an effective amount of alginic acid or alginate, of an effective amount of at least one compound producing a release of carbon dioxide in an acidic medium, and possibly at least one alginic acid cross-linking agent, to prepare a composition suitable for forming, after oral administration to a human or animal individual, a gellified raft floating on the surface of the gastric contents of said individual.

14. The composition according to one of claims 1 to 12, wherein said preparation based on slippery elm bark is a slippery elm bark powder, an aqueous extract of slippery elm bark and/or a hydro-glycerinated extract of slippery elm bark, advantageously a slippery elm bark powder and/or an aqueous extract of slippery elm bark, preferably a slippery elm bark powder; particularly preferably, the slippery elm bark being slippery elm inner bark.

15. The use according to claim 13, wherein said preparation based on slippery elm bark is a slippery elm bark powder, an aqueous extract of slippery elm bark and/or a hydro-glycerinated extract of slippery elm bark, advantageously a slippery elm bark powder and/or an aqueous extract of slippery elm bark, preferably a slippery elm bark powder; particularly preferably, the slippery elm bark being slippery elm inner bark.
